# EUROPEAN PATENT APPLICATION

(11) **EP 4 628 143 A1**
(43) Date of publication of application: **08.10.2025**
(21) Application number: 24168864.7
(22) Date of filing: 05.04.2024
(51) Int. Cl.: A61M 39/10, A61M 39/16

(54) **ASEPTIC CONNECTOR ARRANGEMENT WITH INNER PLUG, AND FLUID CONNECTION SYSTEM WITH CLIP ASSEMBLY TO UNLOCK RETRACTED OPEN POSITION OF TWO PLUGS**

(71) Applicant: SARTORIUS STEDIM FMT SAS, 13400 Aubagne (FR)
(72) Inventor: Delasalle, Brice, 13400 AUBAGNE (FR); Blake, Florian, 13400 AUBAGNE (FR)
(74) Representative: Plasseraud IP

(57) **Abstract**

The connector arrangement has a connector (1) provided with a plug (15) movable inside a tubular body. A blocking part (12a), clipped around the body (2), provisionally locks a retracted open position of the plug, to retain the plug away from a plug seating surface provided near a front central opening of the connector, where a junction plane extends for a coupled state of the connector (1) with a compatible/identical connector (101). An aseptic attachment structure (5) with membrane (M) initially seals the opening. A fluid connection system (100) with a pair of interlocked connector arrangements allows synchronizing membrane removal with unlocking of the respective plugs (15), by forming a removably clippable assembly including the blocking parts (12a, 12b). Each membrane of attachment structure is secured to the clip assembly (12) to be pulled, radially outward, with this assembly. Return means (20) may push the plugs for having aseptic disconnection.

## Description

### FIELD OF THE INVENTION

The instant disclosure generally relates to fluid management systems in the field of biopharmaceutical applications, including valve components for flexibility of fluid circulation, and more particularly to a fluid handling coupling or connector that allows a connection and disconnection, and to a connection/disconnection system using a pair of such connectors for connecting a fluid pipe to another pipe or to a container.

### TECHNICAL BACKGROUND

In the pharmaceutical, chemical field, that use tubing and assembling of components in the processing and transport for fluids (such as biopharmaceutical fluids), there is often a need for aseptic connection, at least for the first connection. Hoses carrying biopharmaceutical fluid may be commonly used for interconnecting flexibles pouches or similar parts of a circuit. The term "biopharmaceutical fluid", in the following, is understood to mean a product of biotechnology (culture media, cell cultures, buffer solutions, artificial nutrition liquids, blood products and blood product derivatives) or a pharmaceutical product or more generally a product intended for use in the medical field.

When using certain substances, it may be necessary to temporarily guarantee sufficiently sterile conditions after various components (pipes and/or containers) have been connected, while maintaining the possibility of uncoupling the connection when necessary. To this end, it is convenient to use fluid coupling components that can be unlocked by a specific action, and which may selectively block or allow flow of fluid through the coupling.

Generally, fluid connection devices comprise a first male connector (in other words forming a male interface) able to be received in a second complementary connector forming a female interface, which is a simple and well-understood solution. However, a male connector cannot be connected to another male connector, nor can a female connector be connected to another female connector. In the context of assembling a flexible pipe to another pipe or container by means of a fluid connection or any assembling of components of a fluid circuit, there is a need to improve the interoperability of the connectors in order to facilitate the construction of modular biopharmaceutical assemblies.

Document US 10267443 B2 shows exemplary fluidic connections, involving two "genderless" connectors that are functionally equivalent in terms of mechanical coupling. Unlike several genderless fluid connectors, which generally require an axial insertion movement followed by a rotational movement about the axis, fluid connection according to this document is easy allowed with efficient completion of the mechanical coupling, while temporary aseptic sealing membranes can be present until a membrane removal operation to enable fluid circulation.

With this type of fluid connection there is no ability to enable disconnection aseptically. Aseptic coupling devices using a valve member or plug have been also proposed for allowing a disconnection. Document WO 2007133793 discloses a device having a wide annular front surface to create a sterile connection with a mating/complementary aseptic coupling device, creating a junction. A pair of springs is used in association with a valve part, so that a closing state to stop the flow of liquid can be obtained. With the kind of assembling provided in this document, each coupling device can be split in two portions once the closing state is obtained, so that a front portion at the junction side is axially moved and disconnected from a rear portion connected to a pipe. Such coupling device is complex to be assembled, thus not optimizing the amount of material (plastic material) and production cost. Also, handling of the system connection using two (genderless) coupling devices of this type is not user-friendly.

More generally, there is still room for improvement regarding constructions of fluid connectors, to optimize the way of operating with such fluid connectors, in aseptic conditions and/or in user-friendly conditions, with an ability to have an efficient disconnection.

### OBJECTS AND SUMMARY

For improving situation, embodiments of the invention provide a connector arrangement for fluid connection, comprising:
- a connector including a tubular body extending around a longitudinal axis of the connector and a plug movable inside the tubular body, the plug being adapted to seal a central opening of the tubular body; and
- an external blocking part provided with a blocker to retain the plug at a retaining region of the plug, which may be a rear retaining region (away from the front opening to be sealed by the plug), so that the plug is maintained in an open position inside the tubular body,

wherein the connector comprises:
   - fixation means;
   - an aseptic attachment structure including a connector front face and a membrane that is removably attached to the connector front face to seal the central opening while the plug is in the open position;
wherein upon removal of the external blocking part, which is coupled (typically directly or indirectly secured) to the membrane, the plug reaches or has been set in a released state so as to be movable, from the open position, to the closing position (in which the plug is sealing the central opening), the plug being provided with a plug axial surface, for instance located adjacent to or extending at the central opening, so that in the closing position the plug axial surface protrudes axially outside, i.e. beyond the central opening, at a front of the connector.

With such arrangement, with the external blocking part mounted removably on the connector to extract the membrane (thus allowing simultaneous removal of these components), a simple gesture allows suitable release of the front opening and release of the plug, typically with the membrane removed by exerting a radial pulling action (radial pulling of the membrane, typically). A clippable blocking part may be provided, to facilitate such gesture. Moreover, in fluid connection system using two connectors and blocking parts belonging to a same clip assembly, it is allowed releasing two plugs distributed in the two compatible connectors, with one gesture serving for the membrane removal.

Such kind of connector arrangement combines ability for aseptic connection, using the membrane of the aseptic attachment structure to initially release the opening/front opening once the mechanical coupling is obtained (with an interlocking by the connecting members to complete the connection, possibly using a catch or latching means), and ability to reclose this opening at the time of the mechanical disconnection, either with suitable return means biasing the plug, or with any suitable controlling operation to move the plug in its closing position. In other words, the connector can combine the following advantages: aseptic initial state, aseptic connection for a coupled state of connectors, and possibly aseptic disconnection when the plugs are configured to automatically/easily reach the closing position (plugs biased by return-member(s), for instance, or controlled similarly).
When establishing a connection system using the connector, the external blocking part may be thus considered as a first unlocking part to be actuated for allowing movement of the plug, while the plug front surface may act as an abutment part that prevents the plug from reaching the closing position when engaged with a complementary plug or similar interfering part provided in a complementary/compatible connector (in mating state between the connectors).

Besides, an automatic closure mechanism may be involved, which is actuated as soon as separation (disconnection) between two connectors of this type is made. In some options of the connector construction, the displacements of the plug can be controlled at each stage:
- initially, to maintain the plug in a retracted open position thanks to the external blocking part or any similar blocking part that can be displaced or removed from a mounting state (mounted on the tubular body),
- after removal of the blocking part, when the connector is coupled with the complementary connector, by having an abutment configuration early obtained, i.e. obtained before the plug can reach a closing state to stop the fluid circulation, with such abutment configuration possibly corresponding to a front-to-front contact between the respective plugs of the connectors,
- and after separation between the connectors, by having means to simply or automatically drive/bias the plug to its closing position and/or means to block the plug in the closing position to prevent any reconnection.

A flange can be formed peripherally on the tubular body, around a central opening that opens a front of the tubular body. In embodiments, the flange comprises the fixation means, which can be provided with a male connecting member protruding axially from a flange front face. The aseptic attachment structure may include the flange front face and the membrane optionally covers a part of the flange, possibly with each connecting member offset on one side of this structure. The membrane may be removably attached (directly attached) to the flange front face or a front of the tubular body. The membrane can overlap a seal provided at a front face of the connector.

The external blocking part may be a bent piece or having a L-profile. At least one clamping member may be included in the external blocking part, clamped and/or clipped on a rear portion of the tubular body (typically with a rear spacing relative to the flange).
Besides, the fixation means may be provided in the flange for an axial connection, the fixation means allowing front fixation, possibly without any rotation involved. For instance, the connector may allow a connection, with a complementary/compatible connector, without any rotation involved due to indexation in rotation provided by the female connecting member and the male connecting member (forming all or part of the fixation means).

In embodiments, one or more of the following features are provided:
- the external blocking part is removably fastened, for instance clipped, on the tubular body.
- the external blocking part may be configured to be fastened to the tubular body before linking the membrane to a front portion (for instance a radial portion) of the external blocking part.
- the tubular body extends around the longitudinal axis between a first open end and a second open end (front end) provided with the central opening.
- the first open end can form rear end when considering the longitudinal axis extends along a rear-front direction.
- the aseptic attachment structure includes the second open end, the membrane being removably attached to the tubular body to seal the opening provided at the second open end.
- the tubular body is a single piece, including the flange.
- the flange may extend perpendicular to the longitudinal axis of the tubular body.
- the flange is overlapped by a longitudinal intermediate portion of the external blocking part, the longitudinal intermediate portion extending between the blocker and a radial securing portion onto which the membrane is fastened.
- the radial securing portion, in the external blocking part, can extend parallel to a transverse plane perpendicular to the longitudinal axis, for instance allowing all or part of the pull portion of the membrane to extend along/parallel to this transverse plane.
- the radial securing portion is parallel to a junction plane defined by the front flange portion of the connector, a front seal being possibly configured to be define an axial sealing contact that is flush with the flange front face.
- the blocker, possibly including two branches for allowing a clipping and/or clamping action with respect to the tubular body, may extend parallel to the flange (along a transverse plane, perpendicular to the longitudinal axis typically).
- an interior space of the tubular body is accessible via the opening (for passage of fluid), the plug, possibly including openings that open toward the central opening in the open state/position, being movable in the interior space, parallel to the longitudinal axis.
- the plug is movable, for instance slidably movable, between a distal position away from the second open end (distal position allowing a fluid to flow through the second open end), and a proximal position in which the second open end is engaged by the plug to seal the opening and allow an aseptic disconnection of the connector.
- the plug may have a sealing position inside the interior space when reaching the closing position, for instance by defining an annular sealing contact with a seating surface that tapers with a tapering direction that is at the opposite/goes away from the first end of the tubular body.
- the plug is longitudinally shorter than the tubular body and entirely extending inside the tubular body in the open position.
- the plug has a closing end portion, which is radially in contact with an inner edge of an inner cylindrical face of the tubular body.
- an annular sealing element may be provided on this closing end portion, circumferentially.
- the closing end portion of the plug may also be axially in contact with an opening rim, either with an inner shoulder or an annular rim portion (beveled edge for instance) facing rearwardly.
- the plug has an axial end forming the closing end portion, without any opening.
- the closing end portion extends between the annular sealing element (carried by the closing end portion, possibly being received in an annular outer groove) and the plug axial surface adapted to extend beyond the opening (outside the interior space of the tubular body), at least in the closing state.

The plug may be provided with radial openings in an intermediate plug section that interconnects a tubular rear portion of the plug, adapted to slide along an inner face of the tubular body, and a plug front end that includes a closing portion, possibly formed as a generally radial portion (with the plug axial surface shifted forward relative to a sealing annular contacting portion defined at the radial portion). Possibly, a fluid interior passage is delimited by the tubular rear portion, this interior passage axially opening radially in at least four radial directions in a chamber of the interior space, which is obtained inside the intermediate plug section. The plug can have a structure made of a single piece, preferably of plastic material, provided with the plugs axial surface and possibly completed with at least one annular sealing member that surrounds the plug closing end.

A fitting may be partly inserted at the first open end of the tubular body, possibly with a collar of the fitting possibly forming an abutment to retain a stationary end of the return member (elastic return member for instance), from the rear so that the return member may extend between the collar and a relief or external shoulder formed on the plug, for instance in the sliding part of the plug.

According to some options, the external blocking part has a clamp part, which may be a rear opposite to a radial front part fastened to the membrane. The clamp part may include a clip or clamping member arranged to engage a partial circumference of the tubular body. Alternatively, or in addition, the clamp part may include a clip or clamping member arranged to engage the plug at the retaining region, while being axially retained by a rim, recessed, slot or groove of the tubular body. This clip may form the blocker.
In some options, the clamp part may be provided with three or four branches, extending from a common base that may constitute the rear end of the external blocking part. The blocking part may be a single piece (blocking clip piece), for instance made of plastic material.

In preferred embodiments, the clamp part may have a C-shape. The clamp part can comprise a first clamping member for outer fixation to the tubular body (to engage a partial circumference of the tubular body) and a second clamping member, possibly under the form of a clip, provided with two branches forming the blocker. Optionally, the two branches are engaged with the retaining region of the plug, which extends axially rearward relative to a sealing annular contact established between an inner face of the tubular body and a circumferential outer part of the plug. A sealing member arranged around a rear sliding part of the plug is typically enabling the annular contact, which is a radial contact against the inner face of the tubular body.
The two branches may directly contact the plug, for instance by engaging inside an outer groove (possibly annular groove) or recess formed peripherally on a sliding part of the plug. The sliding part of the plug may be a cylindrical part (of circular section), a part with an oval or elliptical section being also an option in some embodiments. A seal may be provided or carried by the plug to be interposed between the sliding part of the plug and a cylindrical inner face of the tubular body.

For ensuring stability of a clipping, the blocking part may have two branches, providing an elastic -return effect at ends of the branches, the effect being directed substantially toward the longitudinal axis X (forming a central axis of the tubular body), centripetally in a mounted stat of the blocking part. The two branches may be engaged at two opposite sides of the plug, being partly inserted in the interior space.

In embodiments of the connector, a valve assembly is included in the connector, using a return member, for instance an elastic return member, arranged inside the tubular body to have an end of the elastic return member movable with the plug, parallel or along direction of the longitudinal axis. The plug can be a sprung valve member, maintained axially between the second end of the tubular body and a spring axially resting on a fitting attached (for instance welded) to the tubular body at an annular fastening region of the first open end.
The return member, which optionally may be a single piece, can be a spring or similar elastic return member, for instance. More generally, the return member may be of any suitable structure, so that the return member is biasing the plug toward the closing position, with the return member being possibly a spring (helicoidal spring for instance) of metal or plastic material. An annular barrier may be provided to have a displaceable annular contact between fluid flowing in the interior space and the return member.

The valve assembly provided in the connector includes the tubular body, at least one piece forming the plug, the return member arranged at a rear of a closing portion of the plug, and a sealing element (arranged as piece or part distinct from the closing portion, typically carried by the plug) forming a sealing contact between an annular region where the return member is housed and the closing portion. The valve assembly may also include a fitting of tubular shape (with an annular section) to retain a rear end of the return member, i.e. an end of the return member, which is distal and typically facing at the opposite from the central opening.
The tubular body may define, for instance at an annular portion delimiting the central opening, an annular seating surface in contact with the plug in the closing position.

In some embodiment, the fixation means may be provided with one or more of the following features:
- the fixation means include two connecting members, for instance with a radial spacing between the two connecting members.
- the two connecting members comprise a female connecting member and a male connecting member, preferably distributed on either side of the membrane.
- the male connecting member has at least one size superior to half-diameter defined of the closing portion of the plug.
- the male connecting member is configured to be axially inserted in a complementary female connecting member, along an insertion direction that is parallel (with same orientation) to closing direction of the plug when displaced/set in motion by the return member from the open position to the closing position.
- the connecting members allow the connector to be genderless, the connector being for instance adapted to be connected with an identical connector, except for disposition of the membrane (identical structure may be provided for the tubular body and possibly for the latching means).
- the two connecting members comprise a male connecting member that is protruding axially from the flange front face.
- the two connecting members are offset (radially outward) relative to an annular seal provided in a front groove.
- the annular groove (or front groove) for the annular seal has a longitudinal extension greater than longitudinal extension of a recess, for instance an annular recess, receiving a locking end of the male connecting member of a complementary connector and/or receiving a slider suitable to unlock inserted position of the insert provided in the male connecting member of a complementary connector.
- the flange is provided with a retaining rim or surface to lock an inserted position of an insert passing through an axial access provided in the female connecting member.
- the male connecting member comprises a first insert with a fastening region, preferably under the form of a groove substantially perpendicular to the longitudinal axis.
- the insert or connecting male member comprises an inserting tab that is shifted/offset on a first side relative to the longitudinal axis.
- the insert comprises a longitudinally elongated inserting tab, possibly a flat inserting tab.
- the insert or inserting tab is shifted on a first side relative to the longitudinal axis, a membrane of the aseptic attachment structure being elongated from a covering portion covering the opening (front opening) to a membrane pulling portion.
- the insert or inserting tab is offset laterally relative to the membrane.
- the insert forms an axial tab, possibly provided with a curved (slightly curved for instance) profile.
- latching means are provided in the female connecting member, at the rear of the axial access, possibly in a housing of the flange, to lock an inserted position of a corresponding insert of a male connecting member coupled with the female connecting member.
- the connector is provided with latching means that can be radially movable, preferably toward the longitudinal axis, to engage the fastening region.
- the fastening region may include an outer groove (facing radially outward),
- the outer groove of the fastening region is compatible with a U-section of a latch, with the locked position of the latch corresponding to an engagement of the base of the U-section inside the outer groove, possibly with a return-effect or similar constraint used to maintain the locked position (the latch may be movable, for instance slidable without any rotation. Additionally, the latch may be actuatable like a guillotine except the latch contact edge may be concave to follow shape of curved outer groove formed on the inserting tab).

The insert may be a single protruding member of the connector, which protrudes axially from the front face with a protruding extension greater than a depth (or longitudinal extension) of the axial access provided by the female connecting member. A complementary of shape/circumference may be used for having the insert filling the axial access.
In some variants, the insert is part of a group of parallel inserts that either engage into a single opening or slot forming the axial access, or engage in different openings available on the flange (at the flange front face).

In embodiments of the mechanical coupling part of the connector, the connecting members may include one or more of the following particulars:
- the female connecting member has no axially protruding part, the male connecting member and the female connecting member being distributed on the flange front face, so that the connector is genderless.
- two identical connectors are adapted to be fastened, with each insert (of the male connecting member) radially offset and protruding parallel to the longitudinal axis.
- each axial access of the female connecting member allows insertion along a direction parallel to the longitudinal axis.
- the insert has an insert protruding end (provided with the fastening region) that protrudes beyond a transverse flange wall that surrounds an annular seal carried by the flange around the opening (central opening at the front of the connector).
- the insert fastening region may be facing radially outward when forming of a groove or similar relief part, to be engaged by a part of the latching means that is movable along a radial direction.
- the insert, which may have a beveled free edge or a slanted/thinned edge, may slide inside the axial access of the female connecting member until pushing radially outward the latch or latching means that are movable in the housing or recess provided peripherally in the flange.
- a locking is obtained when a blocking bar/part of the latching means is engaged between two reliefs of the insert or inside a groove of the insert.

In the connector, a seal may extend around the longitudinal axis, surrounding the central opening. An interior space of the tubular body is accessible via the (central) opening. The tubular body may carry a seal of annular shape in a front groove (of the flange) interposed between an inner annular wall of the flange delimiting the central opening and a flange intermediate wall overlapped by the insert of the connecting male member.

The external blocking part can be provided with an axial pin protruding beyond a junction plane defined at a front face of the flange (flange front face). The external blocking part may include a radial contact portion that extends perpendicular to the longitudinal axis (thus along a transverse plane), a part of the membrane, distinct from the covering potion of the membrane, resting on (thus being in contact with) a front surface of the radial contact portion. In some options, the external blocking part may be arranged with one or more of the following features:
- in a connection system involving the connector arrangement and another complementary connector arrangement, the external blocking part in the complementary arrangement can be turned 180° as compared to the external blocking part mounted on the connector (to follow position of the associated membrane).
- the external blocking part may form a partial sleeve.
- the external blocking part may be a single piece (of plastic material and/or including molded plastic material).
- the external blocking part may form a partial sleeve.
- the external blocking part is overlapping the flange on a first flange side.
- the external blocking part is adapted to be in contact with the flange, for instance with an external side wall of flange, on a first flange side.

In some embodiments, the connector has a coupling part formed at least partly by the flange, forming a first insert involved in mechanical connection to obtain a fluid connection. The flange delimits a housing or peripheral region extending around the interior space, the axial access forming an access to the housing or peripheral region and allowing insertion of a second insert of same structure as the first insert.
The second insert can include a fastening region, the second insert being formed in a complementary connector that is suitable to be coupled to the connector for enabling the fluid connection.

The connector is provided with latching means that are movable relative to the flange. A retaining rim or surface, forming all or part of latching means, can be provided to allow a locking. The retaining rim or surface, forming all or part of latching means, can be:
- arranged inside the housing or at the peripheral region; and
- configured to retain the second insert in a locking configuration of the fluid connection by engaging the fastening region of the second insert.
For instance, the locking configuration can be obtained, automatically, when the second insert is in the inserted position and positioned inside the female connecting member via the axial access. The first insert may comprise an inserting tab that is offset on a first side relative to the longitudinal axis, the latching means being peripherally mounted, around the interior space, and being radially movable, preferably toward the longitudinal axis, to engage the fastening region.

In some embodiments, the latching means include a piece distinct from the flange and are operable by a manual actuation using an external actuator or push-button arranged to externally cover the flange and/or radially protrude outward relative to the flange.
Optionally, the manual actuation causes an interfering portion of the latching means to be moved radially away from an axial direction/insertion axis passing through the axial access of the female connecting member.
The external actuator may be pushed, typically radially inward, or similarly actuated. The external actuator may be pushed along a direction which is perpendicular to an extension of the membrane (with extension of the membrane possibly being identical to a direction for pulling the membrane when establishing a fluid connection using the two connectors).

The latching means may be constructed as a single piece that is configured to slide in the housing. The external actuator may be integral with the retaining rim or surface or is operatively coupled to the latching means so that a displacement of the external actuator causes release of the engagement between the retaining rim or surface and the second insert. The external actuator can unlock the fluid connection that has been allowed after the removal of the membrane. The external actuator can be set in motion by a pushing, for instance a radial pushing. The external actuator or push-button can be pushable at a second flange side that is perpendicular to the first flange side.

When having a housing (or emplacement) in the flange for housing an insert end of the male connecting member (the housing possibly also housing a separate latch), such housing may extend peripherally around the fluid passage delimited by the tubular body. In some options, the housing forms a slide extending generally within a transverse plane perpendicular to the longitudinal axis. The housing can house the latching means constructed as a slider (locking slider).
In other options, the insert may be provided with a flexible member that has an abutment surface or relief. The insert with the flexible member can form all or part of the mal connecting member. A U-shaped slot or any suitable separation or reduction of thickness may be provided in the male connecting member to separate the flexible member from a remainder part of the insert that may include a free end of the insert.

When the retaining rim or surface is included in a (preferably single-piece) locking slider distinct from the tubular body and inserted in the housing, the locking slider can be configured to perform the retaining of the second insert at the fastening region of the second insert. When the retaining rim or surface is included is a stationary abutment rim or surface provided in the flange, insertion of the male connecting member can be a clipping, with the flexible member engaging the retaining rim or surface, once inserted in the housing. An unlocking/unclipping of the flexible member may be obtained by sliding a unlocking piece engaged inside the housing to engage, for instance with a radial pushing on the flexible member performed when inserting the unlocking piece in the housing along the clipping region.

In some options, the locking slider, slidably mounted in the housing, is annular, the retaining rim or surface being at an end of the locking slider at the opposite from another end provided with an external actuator. Typically, the end with the external actuator is pushable along a first direction, which is an unlocking direction opposite to an elastic return force provided by the locking slider.
In the locking slider, two flexible tabs, possibly separated by a slot, can be provided at a radial location interposed between the tubular body and the external actuator.

In options using a locking slider as latching means, the flange may be provided with an external side wall having a radial opening through which an external actuator can drive an unlocking of the connection. The following features may be provided:
- the flange has an external side wall that is locally provided, preferably in a corner of the flange, with a side wall opening so that an abutment part/member of the external actuator protrudes radially inward and/or engage the latching means through the side wall opening.
- the external side wall is forming an outer frame surrounding a recess, with the latching means housed in an interior volume of the outer frame, possibly of rectangular shape.
For preventing accidental release of the latch/latching means, a return effect provided by a resilient portion (of the latching means or of a spring biasing the latching means) may be involved for maintaining an engaged position of the latching means with respect to the second insert (of the complementary connector in a connection state) that is
- inserted through the axial access; and
- arranged to protrude inside the housing (at the flange side opposite from the connection side).

In some options using an external actuator distinct from the locking slider, the external actuator may be provided with an abutment member. A slanted surface is provided in at least one amongst the abutment member and the locking slider, the slanted surface being configured so that movement of the external actuator along the first direction causes the locking slider to be driven along the unlocking direction.

In some embodiment, the plug is slidable on a sliding guide provided with an abutment surface, so that the elastic return member biases the plug along a given direction of a biasing force, the plug being configured to be axially blocked by engagement of at least one relief with the abutment surface when the plug is in the closing position, so that (once the closing position is obtained) the plug is prevented to be pushed against the biasing force exerted by the elastic return member. This allows an anti-reconnection effect, which is of interest for a single-use application.
Optionally, the connector may have a valve assembly combining the plug, the return member and the locking mechanism with the abutment surface and the relief (possibly with this relief carried by the plug or by a part movable with the plug). Such connector may be used in any kind of fluid connection system requiring a valve assembly for efficiency of the disconnection, independently from the specific way the initial release of the plug is performed, for instance in a connection system that is provided without any membrane.

In a connection system involving two connectors that each have the above identified features (i.e. with another complementary connector), a clip assembly or sleeve assembly may be obtained by coupling the external blocking parts each fixed on a respective one the two connectors. The external blocking parts are extending on a same side, due to a 180° difference in the disposition of the membrane and the sleeve part in the other complementary connector. The clip assembly can form a bridge component overlapping the two coupling parts (the flanges in particular) of the two connectors.
In the connection system, obtaining the fluid connection (circulation of liquid typically) previously requires removing the membrane(s) with the clip assembly (with removal of each membrane for instance, when using two structurally identical connectors), while the disconnection requires unlocking the fixation means, typically using an external actuator or push button, typically for each connector.

In some embodiments, unlocking may correspond to a removal of the axial abutment, by displacing one amongst:
- the latching means (with movement along the unlocking direction),
- and a flexible lug/part provided in the insert cooperating with the female connecting member.

According to an aspect, embodiments provide a connection system comprising a first connector arrangement with a first connector and a second connector arrangement with a second connector, which are each a connector arrangement as above defined, the connection system allowing fluid connection in a coupled state obtained by coupling parts distributed in the flange of the first connector and in the flange of the second connector, using the fixation means to obtain a front-to-front or flange-to-flange connection between the connectors that have same longitudinal axis in the coupled state (a junction plane being possibly obtained, for junction of the front face of the respective flanges of the connectors).

In connected state of the connection system, the external blocking part of the first connector and the external blocking part of the second connector are:
- each provided with a blocker engaged inside the tubular body to retain the corresponding plug in a retracted open position inside the tubular body (thus, with each plug retracted in the respective connectors, away from a junction plane),
- linked to form a clip assembly, the clip assembly extending parallel to the longitudinal axis;
- configured to be simultaneously removed when pulling radial portions of the membranes, which are overlapping radial portions of the membrane interposed between the external blocking parts and offset relative to a covering region where each membrane axially covers a corresponding central opening.

In the connection system, the plugs are each provided with one or more openings and are biased toward the junction plane of the connection system by return means, so that in the coupled state, the plug axial surfaces are configured to be in mutual contact as soon as the membranes and the clip assembly (have been removed, without preventing fluid to flow between the one or more openings of the plug of the first connector and the one or more openings of the plug of the second connector.

The clip assembly may include a radial graspable portion (possibly perpendicular to the length of the clip assembly). The radial portions can extend parallel to the radial graspable portion. In the clip assembly, each external blocking part may include a first bending area, radially joining the clamp part. A second bending area is provided in a L-shaped section that includes: a half of the radial graspable portion and a longitudinal intermediate portion that overlaps the flange.

In some options, the first and second connectors have identical structure except for orientation of the membrane, so that once the membranes are removed, the first and second connectors are identical, for instance with only a difference of 180° in angular position (around a transverse axis), in the coupled state. Accordingly, the radial portions of the membranes extend in same direction in the coupled state.

An annular contact is provided, typically at the junction plane (after removal of membranes) between two annular seals, each of the two annular seals being carried in the corresponding connector to be present on a flange face, so-called flange front face, that can be opposite to the housing (housing in which the latching means extend).

According to another aspect, a connector arrangement for fluid connection is provided, with a plug that is movable inside a tubular body to reach a closing state near or at a front opening of the tubular body, with the following provisions:
- the plug is movable (released) once an external blocking part provided with a blocker (to retain the plug at a retaining region of the plug), has been removed, being understood the retaining region may be a rear retaining region (away from the front opening to be sealed by the plug), the plug being movable from an initial open position and being adapted to be maintained in an open position inside the tubular body when the connector of the connector arrangement is coupled with a compatible or identical connector;
   and
- a retaining part, for instance under the form of one or more flexible clip(s) or tab(s), is provided in the plug or in a movable part (in a valve assembly) that comprise the plug, so that in the closing state, the retaining part is engaged axially on an abutment surface that is stationary, inside the tubular body, whereby the plug is prevented from returning back away from the front opening: the closing state is a final state for the valve assembly, which is no more openable.

The connector arrangement can comprise:
- a connector including a tubular body (typically extending around a longitudinal axis of the connector) and a plug movable inside the tubular body, the plug being part of a valve assembly that also comprises a return member (preferably an elastic return member) arranged inside the tubular body, biasing the plug toward a closing position;
- an external blocking part, removably mounted on the tubular body, provided with a blocker to retain the plug at a retaining region of the plug, which is preferably a rear retaining region, so that the plug is maintained in an open position inside the tubular body; and
- a locking mechanism to block the plug when the plug reaches a closing position;
wherein the connector comprises:
- fixation means, for instance with a flange formed peripherally on the tubular body around a central opening at a front of the tubular body, the flange comprising the fixation means (possibly provided with a male connecting member protruding axially from a flange front face); and wherein the plug is configured to be:
   - released, upon removal of the external blocking part, to be movable to the closing position in which the plug is sealing the central opening,
   - and then blocked by the locking mechanism that includes a connector stationary part that forms a resting surface for the return member and said abutment surface engaged by the retaining part.

In some options, the connector stationary part is tubular or provided with a cylindrical outer face and the return member is a spring radially interposed between, the tubular body and the connector stationary part, for instance with the return member surrounding the cylindrical outer face of the connector stationary part. The connector stationary part can include an inserted portion of an end fitting that is mounted at an end of the tubular body.

By removal of the external blocking part with two connectors interlocked/coupled by the fixation means, each plug can only move forward (toward a junction plane), due to the configuration of each valve assembly that comprises the return member and the locking mechanism preventing the plug to be pushed rearwardly to a retracted position.
With such kind of valve assembly, a membrane can also be used, being removably attached to the connector front face to seal the central opening while the plug is in the open position. The external blocking part is coupled (typically directly or indirectly secured) to the membrane.

In some options, the valve assembly may be provided with:
- at least one tab that extends longitudinally rearward, at an axial end of the plug (opposite to the plug axial surface/to the front opening), said tab being a part of the locking mechanism.
- a connector stationary part that forms a sliding guide for a cylindrical portion of the plug.
- an annular groove provided circumferentially on the connector stationary part, preferably at the region forming the sliding guide for the cylindrical portion of the plug.
- one or more reliefs, carried by the plug and/or by a movable end of the return member coupled to the plug, configured to engage the abutment surface.
- one or more reliefs, configured to engage the abutment surface, are provided in a clip or in clips (of the locking mechanism) that are longitudinally movable with the plug.

Of course, a connection system, comprising a first connector arrangement and a second connector arrangement as above defined or possibly without the aseptic attachment structure and/by including another kind of blocking parts to be coupled to the connectors, can be provided with a valve assembly optimized to provide an anti-reconnection function, for instance of the type as just above recited.

According to an aspect, embodiments of the invention provide a method of assembling a fluid connector, the method comprising:
- inserting a plug inside a tubular body via a first open end of the tubular body, which is a tubular body rear end;
- clipping an external blocking part around the tubular body and engaging, on a retaining region of the plug, a blocker that is integral with the external blocking part, to retain the plug in an open position inside the tubular body;
- covering (axially covering) a second open end of the tubular body, which is a tubular body front end, by a membrane, in order to seal a central opening of the tubular body and form an aseptic attachment structure, at least one connecting member being provided on a flange formed peripherally on the tubular body, the at least one connecting member being offset relative to the membrane, the membrane extending radially, to provide a pull portion, along a first transverse direction that is perpendicular to a longitudinal axis passing through the central opening;
- coupling the external blocking part to the membrane, so that a pulling action for removing the membrane can simultaneously drive an unclipping of the external blocking part;
wherein the plug is mounted slidable inside the tubular body so that upon removal of the external blocking part by the unclipping, the plug reaches a released state to be movable, from the open position to a closing position.

With such method, a connector may be obtained with a simple mechanical interface combined with an aseptic structure with membrane, allowing simple, possibly quick/automatic closure by the plug. In the closing position, the plug is sealing the central opening and a plug axial surface of the plug protrudes axially outside beyond the central opening. The plugs axial surfaces, provide a simple way to have an interference in connected state, so that further forward mobility (toward the flange front face corresponding to the junction plane) is available at the time of disconnection. Therefore, for a connection system using two connector arrangements, junction at the junction plane between the flanges is preventing the plugs to properly move to their closing position, while disconnection may allow or cause the plugs to be moved forward to seal the central opening. A return member may be provided if automatic and quick closure is wanted without any operation other than the mechanical disconnection using the connecting members.

The membrane is made of flexible material. With the method, the connection system may be provided in a fastened state, i.e. with mechanical fixation by the fixation means performed around the seals provided on each front side/flange front face. The membranes, distributed on the two flanges, are thus sandwiched between the flanges and between two radial portions of the clip assembly (each respectively being included in one of the external blocking parts). Fluid connection is performed in a contacting state of the plugs, in which the plugs are axially in contact one with each other to prevent a further move through the central opening.
Disconnection (with axial spacing between the flanges of the two connectors) is not available before the removal of membranes, due to presence of the clip assembly that also prevents disconnection.

When having a return member associated to each plug (possibly an elastic return member), mechanical disconnection may immediately cause the closing for each connector, due to the displacement of the plugs through the central opening, with a sealing action (by an annular sealing contact obtained only when a free end at the closing portion of the plug is extending/protruding outside, beyond the central opening.
The tubular body may form an outer part of the fluid connector, possibly covering the return member that surrounds an annular end of the plug that is opposite to the closing portion of the plug. The return member may be also radially interposed between the rear end of the tubular body and a male member of a fitting fastened to (and inserted through) the first open end of the tubular body, which is opposite to the central opening provided at a second open end of the tubular body.

According to an aspect, it is provided a use of a clip assembly in the connection system as above proposed, the clip assembly being:
- preliminary clipped around each of the first connector and the second connector, to simultaneously retain the plug of the first connector and the plug of the second connector in a position distal from a seating region that is surrounded by at least one annular seal that is adjacent to the junction plane;
- arranged to pinch the membranes at the graspable radial portion, where the external blocking parts are mutually rigidly fastened.

An interlocking may be provided to ensure that each of the external blocking parts remain mutually secured or not separable before a full removal of the membranes. More generally, unclipping of the clip assembly simultaneously causes:
the membranes to be removed by a radial pulling,
and the two blockers (longitudinally spaced and distributed in the two blocking parts) to be disengaged from the plugs.

To allow mechanical connection/disconnection, by means of the two flanges, a locking and unlocking action is obtained using:
- a given clipping part in the first connector;
- a given clipping part in the second connector;
each clipping part being driven by a corresponding external actuator that is available beyond a side or an external side wall of the flange.
Whatever the shape of the external actuator and the number of piece(s) involved, the external actuator may be operatively coupled to latching means so that, when the connector is coupled to another connector (analogous connector or identical connector with an interlocking at a first insert of the connector and at a second insert of the other connector, using the female connecting members and associated latches/latching means), a displacement of the external actuator causes release of the engagement between the latching means and the second insert, whereby the external actuator can unlock the fluid connection.

The connectors are compliant with a simple way of establishing a fluid connection, while securing a synchronized unlocking of the plugs, which can be also arranged each with an identical return member to be in the closing state.

### BRIEF DESCRIPTION OF THE DRAWINGS

The figures of the drawings are now briefly described.
Fig. 1 is a perspective view of an exemplary fluid connector arrangement before use, with a membrane covering a connector front opening.
Fig. 2 is a longitudinal cross-sectional view of another (complementary/compatible) fluid connector arrangement, here including same connector structure as in Fig. 1, illustrating an exemplary retracted position of an internal plug extending inside a tubular body forming the connector front opening.
Fig. 3A shows a connection system with the two connector arrangements, respectively illustrated in Fig. 1 and in Fig. 2, coupled with each aseptic attachment structure still present, while a clip assembly is obtained by combining the external blocking parts.
Fig. 3B is a detail, in a longitudinal cross-sectional view, of an exemplary position of the plugs such as in the connector arrangement of Fig. 3A, before removal of the membranes, showing an embodiment that does not require a significant plug displacement after removal of the membranes.
Fig. 4 is a perspective view of the fluid connector arrangement of Fig. 1, with a rear part not illustrated, to show an exemplary integration of a locking slider retained by a flange and suitable to lock and unlock position of an insert provided in a male connecting member protruding from a complementary connector arrangement.
Fig. 5A is a perspective view of a locking slider of one piece construction, serving as latching means, here in an option with an external part and a flexible return part provided on a same side of the locking slider.
Fig. 5B is a cross-section view showing the inside of the flanged part of the connector arrangement, with a slide for the locking slider of the type shown in Fig. 5A.
Fig. 6A is perspective view of another embodiment for a locking slider of one piece construction, serving as latching means, here in an option where the locking slider may be pushed by a separate actuator.
Fig. 6B is perspective view of a coupling part included in a connector arrangement that is provided with external actuator suitable to cooperate with the locking slider of Fig. 6A.
Fig. 6C illustrates, with a transverse cut, a flange providing an annular recess housing the locking slider of Fig. 6A, with a radial opening provided in an external side wall of the flange to allow an abutment member of the external actuator to push the locking slider, here along a direction different from pushing direction of the external actuator.
Fig. 7 is a longitudinal cross-sectional view of a connection system including two identical connectors after separation of the clip assembly with the membranes (not shown) and in disconnected state where the plugs are each biased to a closing position, thus preventing contamination in each of the two connectors.
Fig. 8 is a detail view of another embodiment for the male and female connecting members, involving a flexible member carried by the insert of the male connecting member, so that the locking and unlocking actions are a direct clipping and unclipping of the insert.
Fig. 9A is a longitudinal cross-sectional view of a fluid connector arrangement, which can be similar to the arrangement of Fig. 1, with implementation of a mechanism preventing a reconnection.
Fig. 9B is a longitudinal cross-sectional view of the fluid connector arrangement of Fig. 9A, with the plug in closing state and a abutment engagement provided at the rear of the plug, to prevent a return of the plug.
Fig. 10 is an exploded perspective view of a connector of a fluid connector arrangement such as illustrated in Figs 9A-9B.

### MORE DETAILED DESCRIPTION

A detailed description of several embodiments of the invention is provided below, accompanied with examples and with reference to the drawings.

In the various figures, the same references are used to designate identical or similar elements.

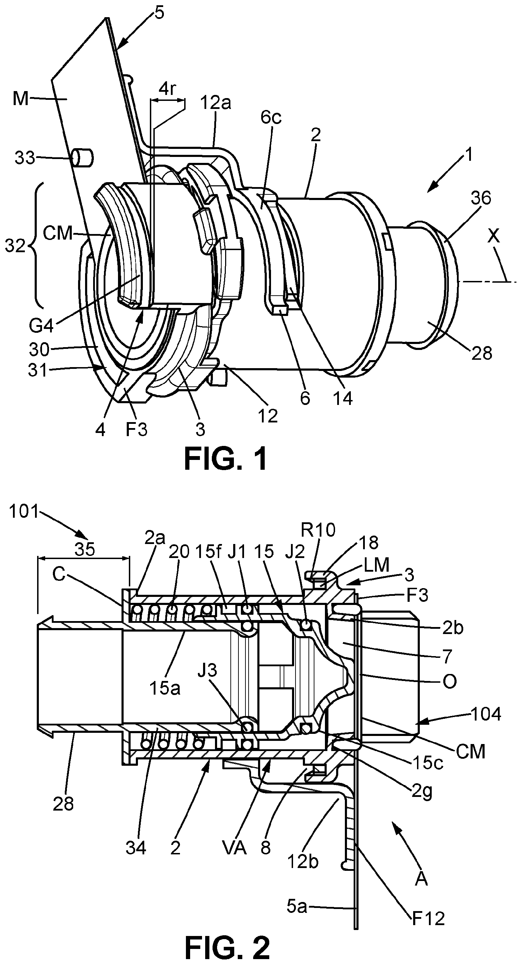

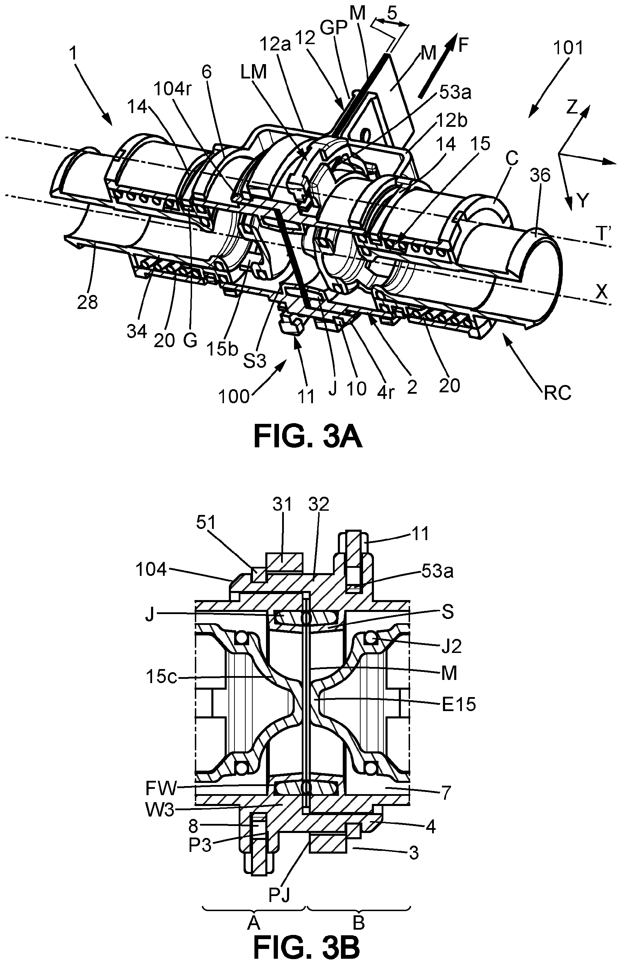

Referring to Figs 1-2, it is shown an exemplary construction of a connector arrangement provided with a fluid connector 1, 101 having a central opening O initially covered by a removable membrane M of an aseptic attachment structure 5. The arrangement provides a fluid passage, along a longitudinal axis X of the fluid connector 1, 101 that may be a central axis of a tubular body 2. The arrangement can be considered as sterilized, thus being treated by suitable sterilization technique, including gamma irradiation, E-beam, ethylene oxide (EtO) and/or autoclave technologies. The fluid connector 1, 101 comprises a main body, which is the tubular body 2, suitable to delimit an interior space 7. The tubular body 2 can delimit a fluid passage of constant section, possibly with an inner cylindrical surface for delimiting the interior space 7.The fluid passage delimited by the tubular body 2 can be rotationally symmetrical about the longitudinal axis X.
As apparent in Figs 2 and 3A-3B, a plug 15 is also present inside the tubular body 2 for enabling a closing operation, which is typically performed when the connector 1, 101 is not coupled with another compatible or identical connector 101, 1.

The membrane M may have a closing portion, here called closing member CM, and a radial extension portion, including or consisting in a pull portion 5a. The radial extension portion may radially extend from an annular margin part of the closing member CM, where the membrane M adheres to an annular seal and/or an annular surface delimiting the central opening O. This opening may be a central front opening O, allowing the membrane M to extend substantially planar and parallel to a front surface F3 of a flange 3.
More generally, the membrane M may be pullable to be removed. The connector 1 can have a flange 3 that extends peripherally around a cylindrical part of the tubular body 2. The removable membrane M prevents bio-contaminants and/or fine particles from passing through. The membrane M may be porous for gas.
Circulation of fluid is allowed via the interior spaces 7, after removal of the membranes M of the connectors 1, 101 that are coupled. After such removal (as described later; see arrow F in Fig. 3A) the plugs 15 interact mutually to prevent closing state to be reached, as long as the connectors 1, 101 remain coupled.

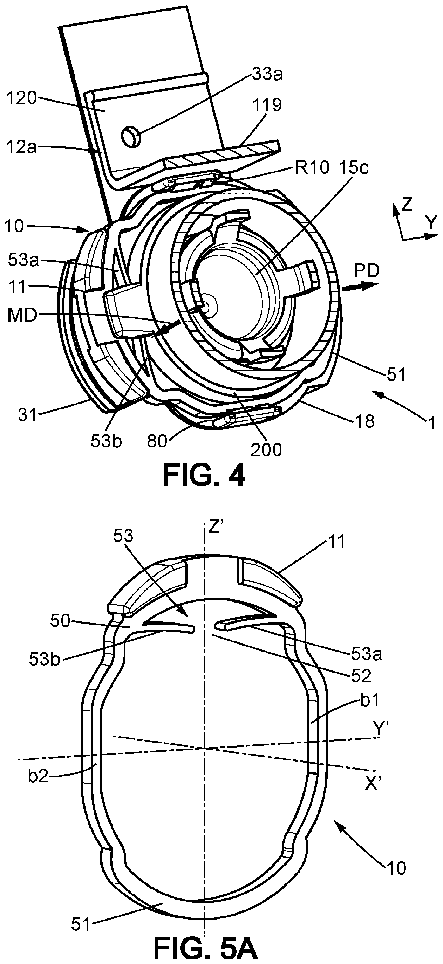

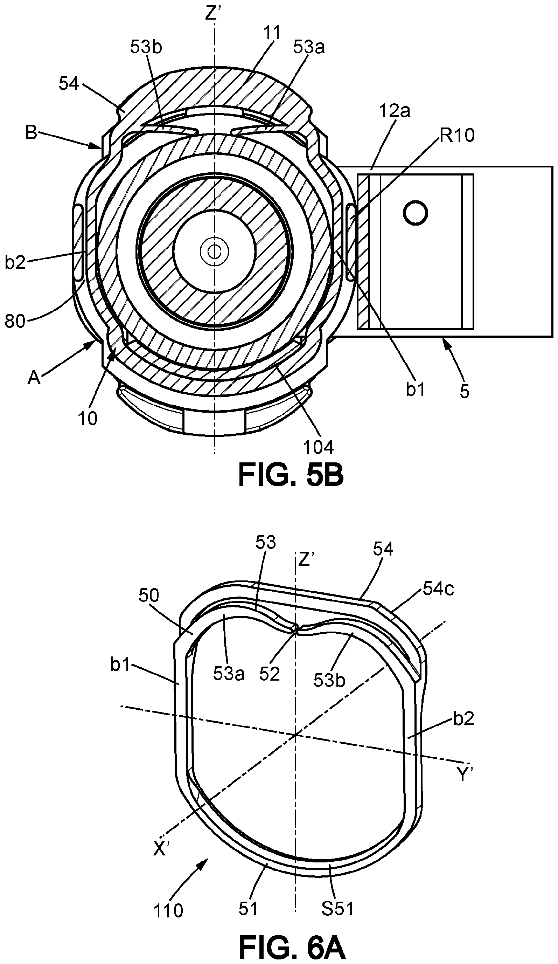

The flange 3 may be included in the tubular body 2 due to a one-piece construction (molded piece preferably), typically using plastic material such as any suitable rigid thermoplastic material. As shown in Fig. 1 the tubular body 2 in the connector 1 may extend around a longitudinal axis X, while the flange 3 formed peripherally on the tubular body 2 also extends around the longitudinal axis X. The flange 3 is surrounding a front part of the cylindrical part and is provided with connecting members allowing a front connecting with another connector 101. More generally, the connector 1 or 101 has a longitudinal extension, here a longitudinal axis X, and the tubular body 2 may be provided with:
- a rear end 2a (at the opposite from the flange front face, as illustrated in Fig. 5); and
- a front end 2b with a central opening O.

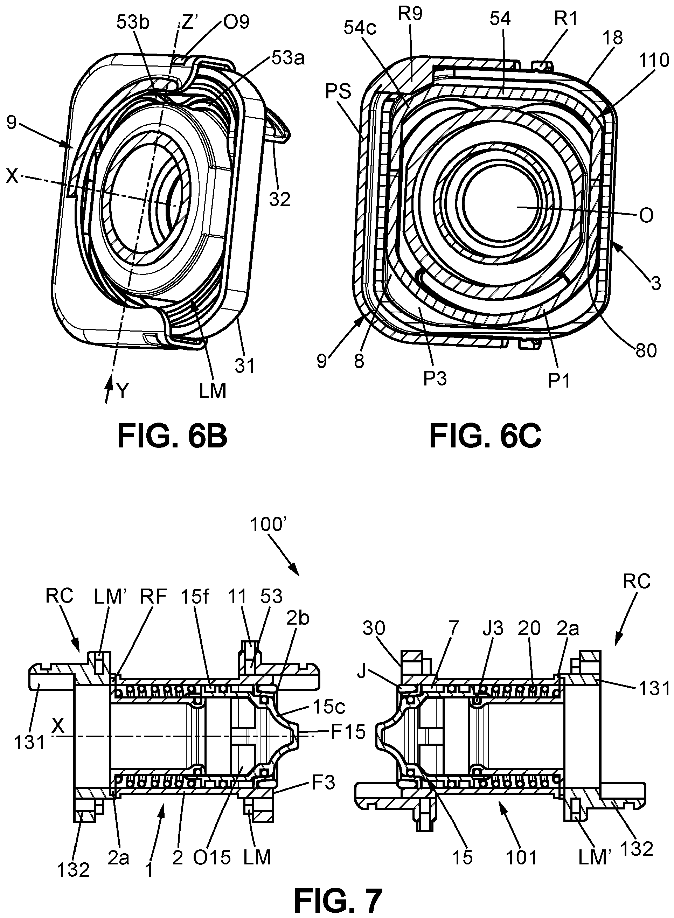

As apparent from Fig. 3A or Fig. 7, the longitudinal axis X may extend, centrally, through the rear end 2a and through the front end 2b. Passage of the fluid conveyed through the fluidic connection using two interlocked connector arrangements 1, 101 is allowed by having in each tubular body 2 an interior space 7 elongated that can house a plugging part, here a sliding plug 15, of a valve assembly VA.

### External blocking part for use in a removable assembly

The plug 15 can extend, initially, between the closing member CM of the membrane M and a return member 20 or similar part forming a contact at a rear end of the plug 15 (at the opposite from the central opening O). The plug 15, slidably mounted inside the tubular body 2 in the interior space 7, is prevented to move forward, possibly prevented to move in any of the front and rear directions, due to an engagement of an external blocking part 12a, 12b with a retaining region belonging to the plug 15. In each connector arrangement 1, 101, the membrane M can be secured to the external blocking part 12a or 12b, for instance using a mechanical connection and/or an adhesive to connect the radial extension portion to a fastening front face F12 provided in the corresponding external blocking part 12a or 12b.

In some embodiments, as reflected in Fig. 1, for instance, a pin 33 or similar relief provided as a protrusion in the fastening front face F12 can serve to interconnect two external blocking parts 12a, 12b, while locally sandwiching the membranes M, away from/with an offset relative to the tubular body 2. A pin receiver 33a, as illustrated in Fig. 4, may be provided for blocking the radial extensions of the membranes, while also obtaining a removable assembly that includes the two external blocking parts 12a, 12b.
More generally, a fastening is established to allow separating the two external blocking parts 12a, 12b simultaneously. The fastening may be obtained by at least one of a shape complementarity, a clamping, a clipping. The fastening may involve at least one relief interfering with the membranes M and/or at least one side fastener that is offset relative to the membranes M.

In each connector arrangement, the connector 1 or 101 is carrying the external blocking part 12a, respectively 12b, provided with a blocker 14 to retain the plug 15 at the retaining region 15f of the plug 15. Each external blocking part 12a, 12b may be bent, so that each blocker 14 extends transversely, typically along a transverse plane, for instance perpendicular to the longitudinal axis X. Two spaced branches 14b are provided in the blocker 14, which are each engaged with the retaining region 15f of the plug 15. This retaining region 15f extends axially rearward relative to a sealing annular contact between an inner face of the tubular body 2 and a circumferential outer part of the plug 15.

Referring to Figs 3A and 7, in a connection system 100, 100' allowing the fluid communication, each external blocking part 12a, 12b is made as a separate part, removable from the tubular body 2 of the connector 1 or 101. The retaining region 15f of the plug 15, for instance a rear retaining region (away/opposite from the central opening O in the connector 1, 101), can be engaged by the blocker 14 via at least one opening or slot provided on the tubular body 2.
In some options, two slots 2s can be provided on two sides, called right and left sides and that are opposite sides along direction of Y-axis such as shown in Fig. 3A. These two slots (transverse slots) thus provide two radial accesses (apertures) for engagement of two branches 14b of the blocker 14 that can be arranged as a clamp.

Referring to Figs 1 and 3A, an exemplary structure of the external blocking part 12a is shown. In this structure, a first clamping member 6 (with two branches 6c for instance) is provided for outer fixation of the external blocking part 12a to the tubular body 2. A second clamping member including two branches 14b can form the blocker 14. The two branches 14 are engaged with the retaining region 15f of the plug 15 to form an axial abutment part extending in a corresponding groove G formed by the retaining region 15f. The retaining region 15f may extend at a location that is axially rearward relative to a sealing annular contact between an inner face of the tubular body 2 and a circumferential outer part of the plug 15, possibly defined by an annular sealing member J1 (typically of compressible plastic material, possibly elastomer) carried by or included in the plug 15.

The external blocking part 12b may be identical to the external blocking part 12a in some options, in particular when the connectors 1 and 101 are of identical structure (thus forming mating connectors of genderless type). While the first clamping member 6 may be arranged around the tubular body 2, the two branches 14b in the second clamping member forming the blocker 14 can directly contact the plug 15. In embodiments, any suitable clamping means can be distributed in the two external blocking parts 12a, 12b to allow the removable assembly to be formed as a clip assembly 12, adapted to be pulled radially outward, possibly using a single graspable portion GP that is the assembly formed by the radial portions 120 (parallel portions) that sandwich the membranes M.
In each external blocking part 12a, 12b, the first clamping member 6 may extend at a rear position relative to radial portion 120, with a longitudinal portion 119 that may form an intermediate portion between the first clamping member 6 and the radial portion 120. The two branches 14b of the blocker 14 may be arched or angled to have a clamping effect. Each of these branches 14b may be hingedly or flexibly connected to common linking part of the external blocking part. The linking part LP may be overlapping a partitioning wall PW section of the tubular body, which is interposed between the two transverse slots 2s. The linking part LP may form a guide, guiding inner faces of the two branches 14b when mounting and when pulling the removable clip assembly 12.

Whatever the way the removable assembly is fastened to the tubular body 2, it allows simultaneously removing the membranes M and shifting position of the blockers 14. The plug 15 is maintained in an open position inside the tubular body 2 when the removable assembly is present. The sealing member J1 may be adapted to slide with the plug, along direction of the longitudinal axis X. Referring to Fig. 3 or 7, the rear end 2a of tubular body 2 can be filled with an inserted portion 34 of and end fitting RC, suitable for connection with a hose or pipe T, T', which is flexible and adapted for a biopharmaceutical fluid. It can be seen that the end fitting RC may have the inserted portion 34, inserted through the tubular body 2 and inside the rear axial end 15a of the plug 15. The inserted portion 34 remains stationary relative to the plug 15, which may be the only movable part in the connector 1, 101.

The end fitting RC can be secured fixedly at the rear end 2a of the tubular body 2, at a rear annular fixation region RF (Fig. 2). The external blocking part 12a, 12b, can engage the tubular body 2 at circumferential region that axially away from annular fixation region RF. A welding may be performed with any suitable end fitting RC. Fig. 7 shows an option where the end fitting RC is provided with a coupling part similar or identical to coupling part A or B of the connectors 1, 101, the coupling part at the end fitting being possibly genderless with a female connecting member 131 and a male connecting member 132. This allows an ease and flexibility of connection with bioreactors, pouches, processing devices, tubing or the like that carry the same kind of coupling parts, preferably of genderless type.
While an inserted portion 34 is here illustrated, options may provide an end fitting without any inserted portion, in some variants. For instance, the return member 20 can be resting on an inner shoulder of the end fitting and the plug 15 may be guided by the tubular body 2, possibly without any contact with the end fitting RC.

A hose nozzle with at least one barb 26 or similar connecting male member (forming an outer coupling part) may be provided at the rear end of each connector 1, 101 or at a rear of the fitting RC in options for connection of a hose or pipe T, T' of flexible material. Due to its position axially spaced from the outer coupling part of the end fitting RC, the external blocking part 12a, 12b, does not interfere with such barbe nozzle or any suitable coupling part provided at the end fitting RC. More generally, it is understood than any other connecting part may be used in the fitting RC, possibly with the connector directly welded on a fastening part of a pouch, a bioreactor or any device used in a circuit where a biopharmaceutical fluid can flow.

Whatever the way the plug 15 is integrated, preferably being inserted from the open rear end 2a, the removable assembly, typically clip assembly 12, can retain the plug 15 in retracted open position. No tool is required for removing the clip assembly 12 or similar assembly with the two blockers 14 in engagement with the respective plugs 15. The external blocking part 12a, 12b can be considered as a locker that must be removed to allow the valve assembly VA to become functional.

### Exemplary structures of the flange in a connector

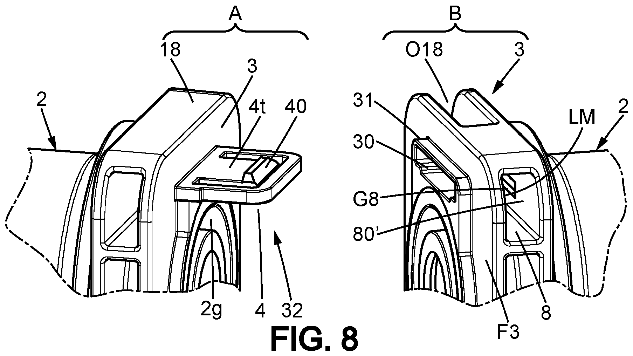

Fig. 3B shows an exemplary way of interconnecting two coupling parts A, B, here with connectors of the genderless type, without any rotation involved for instance. The flange 3, which can be molded from plastic material, can include an intermediate annular wall FW interposed (with a concentrical arrangement) between an inner flange side wall W3 and an external side wall 18, as also shown for instance in Figs 2 and 4. Such configuration may provide a compact flange 3 with:
- a groove of annular shape for a seal J (at the front side), this front groove 2g being arranged between the intermediate annular wall FW and the inner flange side wall W3 that delimits the central opening O;
- and one or more recess(es), with opposite orientation as compared to the front groove 2g, so that a slide 80, 80' is formed.
When having a slide 80, locking of connection may be implemented, for instance using a sliding locker 10 (see Figs 5A-5B). Alternatively, a slide 80' may be provided for allowing a clipping contact or similar fastening to be released (see Fig. 8), using a sliding unlocking device (not shown).

More generally, the flange 3 may be suitable for an efficient interlocking. As shown in Figs 1, 3A-3B, 7 and 8, the flange 3 can be provided with a female connecting member 31 and a male connecting member 32 that is arranged on a different side of the flange, as apparent in the view of Fig. 1 for instance. An axial access 30 is provided at the female connecting member 31, possibly forming a through passage from the face F3 to a recess where a clipping or similar engagement with axial abutment can be obtained to lock a connection between the two mating flanges 3, forming the coupling parts A, B of the connection system 100, 100'.

The aseptic attachment structure 5 is formed by fastening the membrane M to an annular surface of the flange 3 at the flange front face F3, around the central opening O. Such attachment surface is covered by the covering member CM (here a planar member made of the material of the membrane M) that belongs to the membrane M. The covering member CM may have a margin portion that typically contacts and/or extends around an annular seal J received in the groove 2g (front groove) of the flange 3. A complementary portion of the membrane M, possibly of rectangular shape, may extend radially outward from the covering member CM to a pull end part (at the pull portion 5a) that is accessible for the operator.

As apparent in Figs 1-2, the flange front face F3 can be partly covered by the membrane M, with the female connecting member 31 and the male connecting member 32 distributed on either side relative to the membrane M. The male connecting member 32 protrudes from the flange front face F3 and may follow a curved section of the attachment surface and/or be adjacent to the covering member CM. As illustrated in Figs 2, 3A-3B and 7-8, the flange 3 may have a thickness that is superior to a length of the seals J surrounding the opening O. The (female and male) connecting members 31, 32 may be disposed close to the central opening O. Such arrangement can facilitate a manual actuation of the disconnection, with a pushing or any suitable action to be exerted from one side of the flange 3, typically using an unlocking means forming an external actuator 11.

### Interface for removable connection between two connectors

In a connection system 100, 100' combining two connectors 1, 101, at least one female connection member 31 may be involved for an axial retaining effect, thus maintaining a contact between the seals J of annular shape, which may be an axial contact when having connectors 1, 101 of identical or similar structure. The mechanical connection thus uses such female connection member(s) 31 with a corresponding insert provided by the male connecting member that is engaged and locked in properly inserted position. The connection corresponds to a removable fixation, which means a quick disconnection is available, typically requiring unlocking the fixation means. As apparent from Figs 1 and 3A-3B, an external actuator 11, typically a push button, can be provided for each connector 1, 101.
More generally, a movable part can be inserted in a transverse recess/housing 8 or through a radial opening 018 (Fig. 8) to allow disengaging a retaining rim/surface or interfering portion 51 that cooperates with the end/insert of the male connecting member 32. When having a locking slider 10, 110, the interfering portion 51 may be part of the locking slider 10, 110.

In some embodiments, a curved profile is used for an inserting tab 4 (Fig. 1) of the male connecting member 32 and a curved shape can be used as well for the axial access 30 delimited by the female connecting member 31. The size and shape of each profile, for the inserting tab 4 and the axial access 30 may be matching so that the connector 1 can cooperate with an identical connector 101 having same male and female connecting members 31, 32. FIG. 3A and 3B show a connection system 100 with the two connectors 1, 101 coupled at coupling parts A, B that are of identical structure. Each coupling part A or B may include a flange 3 as above disclosed, with the aseptic attachment structure 5.

Figs 2 and 3A-3B illustrate an exemplary inserting tab 104 provided in the coupling part B of a connector 101, which is inserted to protrude at the rear side of the flange 3 of the coupling part A provided in the connector 1. A fastening region 104r (Fig. 3A) of the insert 104 and a fastening region 4 (Fig. 1) of the insert 4 can be engaged to lock the flange-to-flange connection. A transverse guillotine system, compact along the axial direction (along the longitudinal axis X), may allow a interfering portion 51 to be received in the groove G4 formed at each insert (insert tab 4 or 104), possibly with a return effect allowed by an elastic return part 53 as described later. When having a transverse guillotine system, possibly using a locking slider 10, 110 of annular shape to provide the interfering portion 51, external actuation for uncoupling the connection system 100 may be a simple pushing action, exerted peripherally on one side of the flange 3. This allows an easy disconnection to allow axially separating the flanges 3, typically with a pulling of the connectors 1, 101 in opposite directions, while the pushing is exerted to release the respective fastening regions 4r, 104r.
Other variants may be implemented, possibly using a external sliding part that is rigid for interacting with a flexible member 4t of the inserting tab 4, as in the case illustrated in Fig. 8.

Figs 3A and 7 show that a management of fluid circulation in the fluid connection system 100, 100' combining the two connectors 1, 101 can be obtained, using a valve assembly VA in each of the connectors 1, 101. In non-limiting illustrated embodiments, when reaching full/proper insertion of the inserts of the male connecting members 32, a locking of the mechanical connection (flange-to-flange connection) can be obtained, possibly by a clipping involving a flexible part 4t (as in Fig. 8 for instance) and a rigid rim LM' or by using movable or deformable latching means LM, distinct from the tubular bodies 2, that can be arranged at the rear of each flange 3. A locking slider 10 such as illustrated in Figs 5A-5B or a locking slider 110 such as illustrated in Figs 6A-6C may form such latching means LM. Then, after the mechanical connection, the membranes M can be removed to obtain a fluid communication. After removal of the blockers 14, the plugs 15 of the valve assembly VA can move forward to reach an axial abutment state: they are no more in the retracted state with a retaining effect exerted by a respective blocking part 12a, 12b. The plugs 15 are thus less retracted (this displacement possibly being a slight axial movement) and have an opening position, for instance with each plug 15 in abutment at/near the junction plane PJ (at interface between the seals J). Of course, precise area for the plug axial abutment can depend on the design of the abutment surfaces defined by the plugs 15.

### Fluid communication obtained aseptically

In a connection system 100 as shown for instance in Figs 3A-3B, each membrane M can be manually removed with the other one, radially by a pulling action, allow establishing fluid communication aseptically. Arrow F (Fig. 3A) illustrates such manual handling, according to some embodiments. Each pull portion 5a may be available beyond the flanges 3. By pulling at at least one amongst the pull portions 5 (that are in overlapping configuration) of the two membranes M and the graspable portion GP, a simultaneous release of the two openings O can be performed along a first transverse direction Z that is perpendicular to the longitudinal axis X of the tubular body 2. The same configuration may be provided for the connection system 100' shown in Fig. 7 (here illustrated after removal of membranes and after disconnection).

In options with insertion of the inserts or inserting tabs 4, 104 formed by the male connecting members 32, such displacement may be a full insertion, into the axial access 30 in the facing flange, to allow an axial contact between the front open ends 2b of the tubular bodies 2, without any axial interspace at a central region of the connection system 100. The annular seals J (see Fig. 3B) thus can be in axial contact after removal of the membranes M.

Before such removal, it is understood a junction plane PJ is obtained at the interface between the faces F3 of the flanges 3, with the connecting members 31, 32 interconnected. However, the seals J are not contacting (yet) at such junction plane PJ, due to presence of the membranes M. Removal of the membranes M allows obtaining a fluid communication, simultaneously with the axial contact between the seals J.
Without any operator's action after the removal of the assembly 12 with the membranes M, the plugs 15 already enable a flow of biopharmaceutical fluid, by having each an initial position, which corresponds to a retracted state in the valve assembly VA, i.e. with an opening position where each plug 15 has not closing action. Indeed, a closing action of the plugs 15 is neither obtained at the junction plane PJ (now matching with the contact between the seals J), nor at location of the sealing member J2 carried circumferentially to allow an annular contact against the annular seating surface S provided by the tubular body 2. The closing position, which requires a further displacement of the plug 15, can be obtained only in response to a manual operation of the operator, for instance by disconnecting the connection system 100 or 100'.

Fig. 7, with a disconnected state of the connection system 100', shows a closing position of the plugs 15 that can be similar or the same when considering the connection system 100 as in Fig. 3A. It is understood a return member 20, possibly an elastic return member, may facilitate the closing without any latency. After separating the assembly 12 and before any disconnection of the connectors 1, 101, it is understood the plugs 15 may have a suitable configuration, possibly with an interfering action between the two plugs 15, to ensure the fluid communication is established as long as aseptic connection is maintained.
In some variants, one of the connectors used in the fluid connection may be arranged with a plug assembled differently, for instance with a plug that is not biased by any return member but manually controllable, possibly with a closing position driven by a using bayonet system.

With the assembly 12, the connector 1 and the connector 101 can be connected, with each membrane M present along the junction plane PJ, thus forming aseptic mating parts with the coupling parts A, B maintaining the connection. To obtain whole connection system 100, 100', the two connectors 1, 101 may be initially each provided in wrapped condition, thus being in a shipped state to form a sterile connection. Alternatively, the whole connection system 100, 100' can be wrapped. Due to access to the plugs 15 by the blockers 14 of the assembly 12, away from the fluid passage with separation by a sealing member J1, an aseptic connection is established.

While illustrated options show a clip assembly 12 for simultaneous release of the blockers 14, other options may provide a different configuration to allow releasing each blocker 14. For instance, one of the blockers 14 may be provided separately, without any connection to the remainder of the assembly 12. In some variants, a rotation (around longitudinal axis X) or similar driving may be required to firstly selectively disconnect at least one of the blockers 14 (with possibly a pivot connection for a clamping part forming the blocker 14 to be rotated), and then the pulling action is allowed to separate the removable assembly, which is typically a clip assembly 12. For instance, it is understood at least one external blocking part 12a, 12b in the removable assembly 12 may include a blocker 24 arranged to be disengaged or separated:
- before the removal of the other blocker 14,
- or after the removal of the other blocker 14, in a step for separating the assembly 12 from the pair of connectors 1, 101.

### Exemplary valve assembly

Now, referring to Figs 2-4 and 7, a valve assembly VA can be provided in the connector 1, 101. The valve assembly VA has a plug 15 adapted to be displaced in the interior space 7, between a retracted rear position, distal from the central opening O at the front end 2b, and a closing position where the plug 15 is in annular sealing contact with an annular rim or any suitable seating surface S provided at or near the central opening O. The seating surface S may be provided in an annular portion, facing radially inward, of the tubular body 2. A plug axial surface F15, which protrudes beyond the central opening O (thus protruding beyond the front face F3) for the closing/sealing position, may be provided in the plug 15.

The plug 15 has a tubular section, possibly guided by the inner face of the tubular body 2, and a front closing portion 15c. As shown in Fig. 2, the plug 15 may extend between a rear axial end 15a, preferably of annular shape to delimit a flow passage, and the front closing portion 15c. A free end E15 of the plug 15 may be provided at the front closing portion 15c, with the free end E15 and/or any suitable plug axial surface adapted to protrude beyond the central opening O, provided the connector 1, 101 having this plug is disconnected. The valve assembly VA can be provided with a return member 20 that is arranged to bias the plug 15 toward the seating surface S. The return member 20 may be energized, thus being in a configuration where release of the plug 15 by removal of the associated blocker 14 and removal of any abutment force exerting in reverse direction causes the plug 15 to be automatically pushed or moved (typical with a sliding) in the closing position.

The plug 15 is also provided with an intermediate portion 15b making a transition between the closing portion 15c that can be a radial portion and an annular section (for instance cylindrical) provided in the axial end 15a that extends annular around the longitudinal axis X. The plug 15 may be a single piece, except one or more possible sealing member(s) J2, at least one sealing member defining an outer circumference at the closing portion 15c. For each connector 1, 101, a sealing member J1 may be also provided in a sliding part of the plug 15, for instance for a displaceable contact against the tubular body 2, thus preventing any peripheral circulation of fluid around the rear axial end 15a to join the central opening O. A seal/sealing member J3 may also be provided on the inserted portion 34 for separating the fluid passage from such peripheral area. The return member 20 may be possibly extend in a peripheral area where no fluid circulates, due to the two sealing contacts provided, respectively on the inner face and on the outer face of the plug 5.
In such configuration, fluid circulation beyond the plug axial end 15a via the openings 015 is only enabled in a plug inside space surrounded by the plug 15 and by the inserted portion 34. Optionally, the radial openings O15 may be distributed at different angular sectors in a same section of the plug 15, without any axial shifting between, these radial openings O15.

The intermediate portion 15b may be discontinuous along circumferential direction, with the adjacent radial openings O15 (as apparent in Fig. 2, 3A and 7) separated by struts or similar linking members. These longitudinal struts interconnect the closing portion 15c to the tubular rear portion of the plug 15, which is a sliding portion adapted to slide along an inner face of the tubular body 2. Referring to Figs 2, 3A and 7, the plug 15 may define a flow passage delimited by the tubular rear portion. This passage may radially open in three or four directions for instance (thus with the flow possibly divided in four sub-flows, when having four radial openings O15).

With such arrangement, in open state of the valve assembly VA, flow coming from the rear can pass around the closing portion 15 that may be of lower cross-section than the rear axial end 15a, or located in a chamber of wide section (possibly wider than a diameter of the central opening O), to finally reach the opened junction at the junction plane PJ.
More generally, with such plug 15 movable to reach the junction plane PJ, dead leg or any significant interspace is limited, possibly prevented.

The valve assembly VA may be assembled using four main components: the plug 15 (possibly including the sealing elements J1, J2), the return member 20, an end fitting RC to retain the return member at a rear side and a cylindrical part of the tubular body 2 that typically surrounds the return member 20. Such configuration may allow the tubular body 2 to be relatively short/compact to facilitate handling, for the unclipping of the clip assembly 12 for instance. No manual operation is needed to control the valve assembly VA, when having a configuration with spring plugs 15 or plugs similarly biased toward the closing position but with two locking levels, typically:
- a first locking level due to the external blocking part 12a, 12b;
- and a second locking level that can be obtained by interference at the free ends E15 or at any plug axial surface F15 that protrudes beyond the central opening O for the closing position, due to a plug-plug axial contact.
Of course the return members 20 may be configured to have opposite actions in a connection system 100, 100' using same or similar kind of valve assembly VA in each of the connectors 1, 101.

Whatever the precise position of the plug 15 for a disconnected state of the connector 1, 101, i.e. after use (and thus after removal of the external blocking part 12a or 12b), the valve assembly VA can be provided with locking means (locking mechanism) preventing a rear motion of the plug 15 toward a retracted position. While Figs 1-7 show a spring forming the return member 20, to have the plug 15 biased toward the closing state (state as illustrated in Fig. 7 for instance), any suitable return member and/or locking means can be used to prevent the plug 15 being moved back. As a result, a leak of fluid may be efficiently prevented.

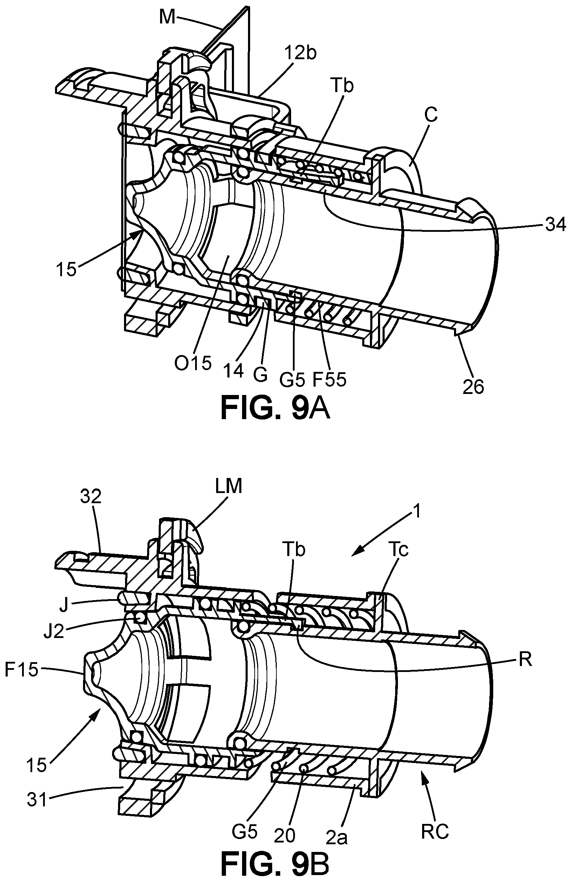

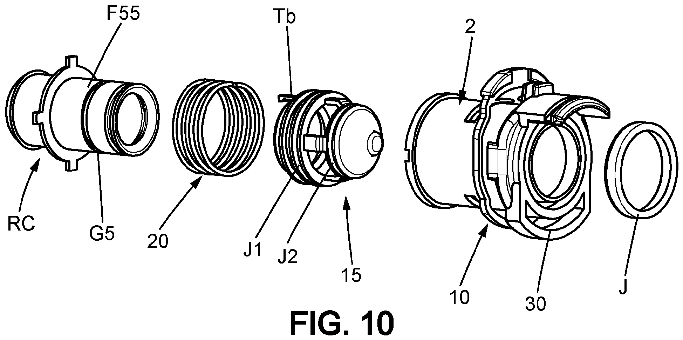

An exemplary locking mechanism is illustrated in Figs 9A-9B and 10, showing the connector 1, 101 can be provided with a plug 15 that:
- can move only forward at the time of disconnection,
- and then, is blocked by the locking mechanism, with a groove G5 (here of annular shape) or similar part used to provide an abutment region, onto which a portion of the movable part of the valve assembly VA is engaged and typically retained.

Referring to Figs 9A and 9B, it can be seen the end fitting RC can form a stationary part of the valve assembly VA. The groove G5 can be provided on such stationary part, here on an outer face F55 of the inserted portion 34 that belongs to the end fitting RC. While a collar C with radial tabs Tc has been shown in this non limiting-embodiment, to allow fixing the end fitting RC, it is understood that any suitable piece or shoulder part can form a retaining stationary part for:
- providing a resting surface (at a first abutment region) for the return member 20 (where a contact is established with an end of the return member that remains stationary in the connector 1, 101), and
- providing the groove G5 or similar relief, possibly of annular shape, in a region (second abutment region) that may be overlapped by the return member 20 and/or the plug 15 at least in the initial position of the plug 15, when the external blocking part 12a or 12b is engaged with the retaining region 15f (provided with the groove G).

When having such locking mechanism, no reconnection is allowed and a connector arrangement for fluid connection can be obtained with: the connector 1 or 101, the external blocking part 12a or 12b provided with the blocker 14 (for an initial retaining effect, at the first abutment region); the fixation means that may be formed on a flange formed peripherally on the tubular body; and the valve assembly VA that includes any suitable plug 15 forming the retaining region 15f and configured to be retained on the groove G5 or similar relief (at the second abutment region), due to a locking mechanism such as above described.

Upon removal of the external blocking part 12a or 12b (typically when already having the connectors 1, 101 coupled), the plug 15 is released:
- to be movable to the closing position in which the plug is sealing the central opening,
- then, to reach the closing position, as reflected for instance in Fig. 9B, while also being blocked by the locking mechanism (at the second abutment region), for instance using a clip or tab Tb (integral with the plug 15) that overlaps the groove G5 and is retained in the groove G5 by a radially protruding relief R of the tab.

Having a combination of the return member 20 and the clip or tab Tb can be advantageous to have the plug 15 being suitably pushed to reach the closing position as soon as the connector 1 or 101 is disconnected. While the illustrated embodiments show a specific face F15 in each plug 15 to allow the plugs to be in axial contact to allow the fluid communication, as reflected in Fig. 9A, other options may involve a peripheral contact (around/externally to the seal J for instance) to prevent the plug 15 to move further forward. Besides, as illustrated in Fig. 10, the parts of the connector 1, 101 can be assembled with use of any kind of external blocker, possibly not associated with a membrane M in some options. The valve assembly VA can be configured with any suitable initial configuration that is unlockable, using an external blocker 12a, 12b to release movement of the plug 15.

The plug 15 can be slidable on a sliding guide, possibly under form of the inserted portion 34 (forming a suitable outer face F55) or any similar guide fixedly carried (directly or indirectly) by the tubular body 2. The sliding guide can be provided with the abutment surface that can be arranged as a shoulder, groove G5 or local protrusion matching with the relief(s) R formed in an anchoring rear part of the plug 15. Each relief R can be included in a resiliently deformable member, such as the tab Tb or similar clip carried by the plug 15 at rear/axially shifted relative to an annular sealing contact plug - tubular body (contact established via the sealing element J1). The relief R may be provided near, adjacent to or at a free end of the clip or tab Tb. The plug 15 can be here made of plastic material and a hinge effect may be provided at a junction with the clip or tab Tb. In some variants, another plastic piece, distinct from the plug 15, may include the relief R that engages the abutment surface formed by the groove G5 or similar fastening region to have the plug 15 blocked.

The return member 20, which may be an elastic return member 20 having a fixed rear end resting on a portion or collar C of the end fitting RC, is mounted inside the tubular body 2, in order to bias the plug 15 along a given direction of a return member biasing force. Besides, the clip or tab Tb may be energized (with a constraint maintained), for instance with the clip or tab Tb flexed radially outward due to interference between, a relief R and the sliding guide, to have an unstable configuration due to the plastic deformation: this deformation can be maintained by a relief contact (radial contact) onto the sliding guide, for instance a contact against the outer face F55, as in the case illustrated in Fig. 9A.

An outer groove G5 can formed circumferentially on the outer face F55, so that a recess region is formed. The outer groove G5 may be located axially between:
- a sealing contact area (typically provided at an end of a cylindrical portion of the sliding guide) for contact with the plug inner face, using the annular seal J3;
- and a collar C of the end fitting RC that defines an abutment for the return member 20.
The outer groove G5 with the recess region allows a the clip or tab Tb to substantially return to its original shape, by receiving the relief R that is slightly displaced radially inward (due to the reverse plastic deformation) to be in axial engagement against the abutment surface that may be form a rear delimitation of the groove G5.

Upon removal of the blocker 14 or any similar portion of the blocking part 12a, 12b involved to initially keep the plug 15 away from the closing position in the connector 1 or 101, the plug 15 can be displaced with the help of the return member 20. Only after disconnection has been performed (separation of the connectors 1, 101), the return member 20 can further push the plug 15 along a predefined stroke, until the plug 15 simultaneously:
- reaches the closing position, and
- remains axially blocked, with the clip or tab Tb preventing any reverse plug motion, the relief(s) R being definitively engaged against the abutment surface.
In other words, the plug 15 is prevented to be pushed against the biasing force exerted by the elastic return member 20. The further pushing of the plug 15 thus happens only once, immediately after the disconnection: then, no access to the interior space 7 via the opening O is allowed (no reconnection).

In some options, the connector 1, 101 can axially maintain a spring or similar elastic return member, forming the return member 20, between two shoulders, one of which is a shoulder part provided on the rear axial end 15a of the plug, the other shoulder being a collar C of the end fitting RC. A barbed end 28 or similar nozzle may optionally be provided as an axial extension of the tubular body rear part, at the rear of the connector 1. In some variants, such barbed end 28 may be replaced by a flanged coupling part such as illustrated in Fig. 7, with connecting members 131, 132, possibly arranged to form a genderless rear coupling part at the opposite from the central opening O.

The inserted portion 34 can bear an annular seal J3 and is suitable to guide the sliding portion/rear end 15a of the plug 15. While only the front seal J3 distal to the collar C is in tight contact with the plug 15 is the closing position, as illustrated in Fig. 2, this seal (with the seal J1 being proximal to the collar C) can be in contact with outer cylindrical face of the sliding portion of the plug 15, when the plug 15 is retracted. The stroke of the plug 15 may correspond to an axial distance lower than axial distance between the seal J3 and a rear end edge of the plug 15 as measured in the retracted position.

### Examples of position-locking system to prevent accidental disconnection

Referring to Figs 1, 2 and 3A, the connector arrangements (with the connector 1 and with the connector 101, respectively) have each an offset radial portion parallel to the membrane M, this radial portion being possibly radially spaced from the tubular body 2. In assembled state of the two connectors 1, 101 (with locked position), the radial portions form a graspable portion GP. The hoses/pipes T, T' or devices may be connected to the connection system 100, 100' obtained with such graspable portion GP provided as a radial outer protrusion of the clip assembly 12.
Accidental disconnection of the male connecting members 32 is prevented by having external actuators 11 arranged in different angular sectors in the connection system 100, 100'.

In some options, the latching means LM, LM' cannot be disengaged, unless being displaced by a suitable sliding part or abutment member R9 (Fig. 6C), which is a part carried or integral with the external actuator 11. Providing the external actuators 11 at different angular sections in the connection system is compatible with use of genderless coupling parts A, B and thus with use of identical structure for the two connectors 1, 101. In some options, the external actuator 11 is included in the locking slider 10, as shown in Fig. 5A for instance.

Referring to Figs 5A-5B, it is proposed a locking slider 10, or any similar annular suitable piece of the latching means LM, possibly having a symmetry axis Z', so that the two straight sliding sides b1, b2 are at equal distance from such axis Z'. This axis Z' can match with Y-direction of the connector 1, 101 in mounted state of the locking slider 10. A slide 80 can be formed in the flange 3 to allow sliding displacement of the locking slider 10 along direction the axis Z'. A pushing direction PD (Fig. 4) may be defined for each connector 1, 101.
The locking slider 10 may have a slot 52 at an end that corresponds to a pushing side of the external actuator 11. The slot 52 is separating two flexible tabs 53a, 53b that may form the return part 53. The flexible tabs 53a, 53b, extending away from a longitudinal axis X of the locking slider 10 (but arranged closer than the external actuator 11) can resiliently engage an outer region of the tubular body 2, as shown in Fig. 5B, thus biasing the locking slider 10 toward a locking position, in which the interfering portion 51 (here opposite to the external actuator 11 along Z'-axis direction) is engaging an inserting tab 4 or 104 to prevent flange-to-flange disconnection.

The locking slider 10 may be formed in a plate or similar single piece. The return part 53, included in this piece, may exert a return effect along direction MD that is, as illustrated in Fig. 4, opposite to pushing direction PD for the pushing applied on a side of the connector 1.

The locking slider 10 may be clipped, using lugs or outer reliefs R10 (Fig. 2) that locally protrude inwardly from the external side wall 18. For instance, the reliefs R10 are arranged/distributed at opposite sides along the Y-direction and configured to allow, after mounting, a further displacement of the external actuator 12, so that a sliding is obtained in response to pushing actuation on a proper pushing surface PS at an accessible end of the locking slider 10.

In some options, as shown in Fig 6A-6B for instance, the locking part or similar latching means LM consists in a locking slider 110 that is sliding along a direction perpendicular to the pushing direction. The manual pushing may be exerted at an external actuator formed as an external part 9, covering the flange 3 on at least one side. Referring to Fig. 6C, it can be seen a housing 8 may be defined around the plug 15, radially beyond the groove 2g for the seal J, so that latching means LM may be carried (preferably inside the housing 8) by the flange 3 that also carries the external part 9. The external part may be slidably mounted on the flange 3. While the external part 9, provided with a pressing/pushing surface PS, has here a structure of a push button with two arms forming two parallel sliding sides for having a mobility on the flange 3, other constructions may be used, possibly using a hinged actuator or any suitable structure with a movable part.

The external part 9 may be C-shaped, U-shaped and/or configured to cover three distinct sides of the flange 3, while being slidably mounted along direction of the axis Z. Possibly, the removable clip assembly 12 may prevent actuation of such external part, in some options. When the external part 9 comprises two sliding arms, the female connecting member 31 and the male connecting member 32 can extend each in an intermediate position between:
- the intermediate wall FW that surrounds the seal J,
- and one of the sliding arms.
Openings O9 may cooperate with outer reliefs R1 of the external side wall 18, with abutment contact of the reliefs against rim(s) of the opening O9, to prevent separation of the external part 9.

Referring to Figs 3A-3B and 7-8, in the connection system 100, 100', each male connecting member 32 is clipped in the housing 8 or similar recess/groove provided beyond the transverse wall of the flange 3 crossed by the inserting tab 4, 104 of the male connecting member 32. The width of each inserting tab 4, 104 may be superior or equal to a width of the flange 3 and at least one bevelling may be provided at a free end of the inserting tab 4, 104, to help in having a guided insertion, simultaneously, of the inserting tabs 4, 104, in opposite directions. The locking is also performed simultaneously and automatically, using for instance same locking slider 10, 110 interfering at an area of the recess/groove. At this area, the axial access 30 opens for the passage of a fastening region 4r, 104r orientated radially outward on each inserting tab end.
The retaining/interfering portion 51 of the locking slider 10 may also have a ramp or bevelled surface S51, intended to facilitate the insertion movement of the male connecting member 32 which must move the locking slider 10, 110 radially outwards until the retaining annular rim (typically rim of a groove G4) has passed beyond the interfering portion 51, which then causes the locking slider 10, 110 to rise in the groove/to move centripetally due to the return forces associated to or integrated to the locking slider 10, 110, possibly using flexible tab(s) 53a, 53b (see Figs 5A-5b and 6A-6C).

For having such locking action, the locking slider 10 is shown as an exemplary piece forming the latching means LM, using an annular shape fitting with the annular shape of the housing 8, possibly using retaining tabs R10 for only allowing a sliding of the locking slider 10. Of course, other structures may form the latching means LM, for instance using a recess arranged only in a given angular sector in the flange 3, where the inserting tab or insert to be engaged extends. This angular sector may correspond to the side where a pushing is exerted on the external actuator 11 or external part 9.

In the connection system, the two connectors 1, 101 are coupled so that the external actuators 11 or external parts 9 (sliding parts) are both pushable from different directions, possibly opposite transverse directions. In the option of Fig. 8, an external part similar to the one illustrated in Fig. 6B-6C, except that this external part slides in a direction transverse/perpendicular to the insert tab 4 to be disengaged. In the option shown in Fig. 6C, the external part 9 can be pushed to have a abutment member R9, provided in a corner region, that converts this pushing into a perpendicular sliding of the locking slider 10.

Stability of the connection system is here obtained by latching means LM that are each stably maintained in the housing 8, in order to avoid accidental disconnection due to accidental removal of the male connecting members 31.
Now referring to Figs 6A-6C, an exemplary locking slider 110 will be described. With an annular shape using four narrow sides (of low thickness), the latching means LM may be formed with optimized use of plastic material. Two parallel arms b1, b2 allow the sliding. The locking slider 110 ensures a combined retaining action and sliding action, around the tubular body 2 The retaining tabs R10 may form means for definitively retaining the locking slider 110. A similar retaining arrangement may be provided for options with the locking slider 10.

In some options, a pair of retaining tabs R10 is provided: one protruding radially inward to block the arm b1, the other one protruding radially inward in opposite direction (transverse protruding direction of the tabs R10, parallel to Z-direction) to block the arm b2, whereby the arms b1, b2 can slide along the Y-direction. The locking slider 10 cannot move along direction of the longitudinal axis X, and cannot move along Z-direction due to sizing/external width of the locking slider 10 along its left-right transverse direction Y', which is parallel to Z-direction in mounted state.

Referring to Fig. 6A, it can be seen that the locking slider 110, or any similar annular suitable piece of the latching means LM, may be an unlockable locking element, provided with an outer part, possibly annular with a symmetry axis Z', so that the two straight sliding sides/arms b1, b2 are at equal distance from such axis Z'. This axis Z' can match with Y-direction of the connector 1 in mounted state of the locking slider 10.
The locking slider 110 may have a slot 52 at an end that corresponds to a pushing side of the bridge portion 54, the slot 52 separating two flexible tabs 53a, 53b. More generally, a deformable portion can be provided, preferably axially offset relative to a contact portion, called bridge portion 54, driven by the external part 9.

Referring to Figs 6A and 6C, the contact/bridge portion 54 may also extend, transversely, at the same end of the locking slider 110 as the flexible tabs 53a, 53b but with an axial offset along Z'-direction. The bridge portion 54 may extend radially farther from the longitudinal axis X and may also be offset along a central axis X' of the locking slider (parallel to X-direction). The bridge portion 54 thus may be rearwardly offset as compared to the elastic return part 53, here provided with the tabs 53a, 53b. Like the locking slider 10, a junction 50 can be provided between the bridge portion 54 (external actuator 11 when considering the locking slider 10) and the arms b1, b2, to have the locking slider 110 formed as a closed loop. For each locking slider 10 or 110, the flexible tabs 53a, 53b may extend each from an arm end to a free end delimiting a side of the slot 52. The interfering portion 51 of the latching means LM, when using a locking slider 10, 110, can be moved radially away from an axial direction passing through the axial access 30 of the female connecting member 31, to allow disconnection.

In advantageous options, the two flexible tabs 53a, 53b can be arranged symmetrically with respect to the X'Z' plane. In addition, the flexible tabs 53a, 53b are arranged (along Z'-direction) under the bridge portion 54. Typically, in mounted state, they are interposed between:
- the bridge portion 54 or the actuator 11,
- and a continuously curved surface of the intermediate wall FW.
In addition, one will note that each of the flexible tabs 53a, 53b has a curvature oriented away from a central region of the bridge portion 54 (curving downwards in non-limiting embodiment of Fig. 10A). Also, as reflected in non-limiting example of Fig. 6A, each of the flexible tabs 53a, 53b optionally has a cross-section or thickness that decreases from its root to its free end (where the slot 52 is delimited).

Referring to Fig. 5B or 6C, the elastic return part 53 may be configured to be in radial contact against the tubular body 2, around a path (in the interior space 7) where the closing plug portion 15 is displaced. For the connection, when an inserting tab 4 or 104 engages on the interfering portion 51 provided in the housing 8 or suitable recess, starting to axially slide on beveled surface s51 provided at beveled front side of the interfering portion 51, a clipping is obtained once the interfering portion 51 reaches the groove G4.
In options without using such locking slider 10, 110, an inserting tab 4 or 104 can include a partly flexible tab 4. As shown in Fig. 8, a slot (possibly U-shaped slot) or any suitable separation or reduction of thickness may be provided in the male connecting member 32 to separate the flexible member 4t from a remainder part of the insert (inserting tab here for instance) that may include a free end of the insert. A cavity or groove G8 may be included in the flange 3, for instance in a bottom portion facing the axial access 30. The free end of the insert thus can engage inside such cavity or groove G8, once the insert is clipped using the flexible member 4t with an appropriate abutment surface or radial relief/lug 40. The insert can be a clip having a rectangular tab shape or any suitable shape passing through the axial access 30 and allowing a retaining effect against an abutment rim of the latching means LM'.

More generally, the flange 32 can be provided with any suitable retaining rim or surface to lock an inserted position of an insert, possibly an inserting tab 4, 104 passing through the axial access 30 provided in the female connecting member 31. The recess or housing 8 may include a transverse slide 80, 80' perpendicular to direction of the insertion direction of the insert to allow a sliding member to have an unlocking action with a release of the engagement obtained with the insert in the recess or housing 8.

Referring to Fig. 7, it is understood that latching means LM2 may be arranged to have a locking in a region of each female connecting member 131 where an insert (of the complementary male connecting member 132) is properly inserted via a similar axial access in a flange carrying the connecting members 131, 132. Typically, the latching means LM2 may include a locking slider 10 or 110 or may implement a clipping arrangement such as illustrated in Fig. 8, possibly also with same kind of unlocking as above described. Accordingly, a connecting device can be obtained with a first coupling part, typically genderless, and with a second coupling part, also genderless and possibly identical in flange structure as compared to the first coupling part (arranged axially at the opposite from the second coupling part).

It is understood that the locking action by the latching means LM, LM' can allow an axial pressure to be exerted on each O-ring or seal J, to ensure a sealing function (the membranes being no more present) is performed around the central passage defined at the openings O. The sealing elements or seals J may have an excess in length to ensure an axial pressure is exerted in such annular contact area, forming an annular junction.

The insertion movement is completed when having a clipping action, using the groove G4 and/or a radial relief/lug 40. Use of a locking slider 10, 110 engaged in the groove(s) may efficiently prevent accidental disconnection, especially when having an elastic-return part 53 that prevents radial displacement of the interfering portion 51, unless the locking slider 10, 110 is displaced along the slide 80 by a pushing action exerted at the pushing surface PS.

The two flexible tabs 53a, 53b may be formed integrally with the locking slider 10, the flexible tabs returning the locking slider 10 toward a first position, referred to as the locking position, with the interfering position being proximal to the longitudinal axis, the fastening region 4r or 104r being retained by the retaining an interfering portion 51, here curved with same profile as the groove G4 provided in the fastening region 4r, 104r. The two flexible tabs 53a, 53b are adapted to flex to allow the plate or similar piece of the locking slider 10 to move to the second position, referred to as the unlocking position.

The bottom wall P3, the external side wall 18 and the intermediate wall FW can form the housing 8, made integral with the tubular body 2. Referring to Fig. 3B or 6C, the external wall 18 can provide two parallel guide portions, parallel to transverse direction Y.
Referring to Fig. 6A, symmetry axis Z' of the locking slider 110 may optionally define a length direction of the locking slider 110, while a particular corner 54c (being exception in a full symmetry of the locking slider 110) formed at an end of the bridge portion 54, on a given side along Z-direction which is the pushing surface side, defines an abutment surface in contact with an offset abutment member R9 provided as an inward protrusion in the external part 9, protruding through a radial opening of the external side wall 18 in the optional structure used in Fig. 6C.

While some of illustrated embodiments show a locking slider 10 or 110 of annular shape, other structures may be provided, for instance using a U-shape of the locking slider with arm end providing the engagement parts to interact with a fastening region 4r, 104r of an insert passing through the axial access 30. Besides in Fig. 8, an external actuating part having a general U-shape may be used for a sliding through the radial opening O18, a maximal sliding position allowing an unclipping of the insert/inserting tab 4.

Whatever the way the unlocking is driven, a pair of arms may be included in a sliding piece having the pushing surface PS. Fig. 6C and Fig. 8 show exemplary circumferences of the external side wall 18, with the opening O18 allowing the appropriate engagement to unlock the connection. Displacement of the external actuator 11 or external part 9, for instance when pushed along Y-direction, causes release of the engagement at the latching means LM, LM'.

### Exemplary operating mode using the two connectors

Using the pins 33 and pin receivers 33a or similar linking part to maintain the external blocking parts 12a, 12b assembled, the connectors 1, 101 may be provided in an interconnected state, with a pair of aseptic attachments structures 5 for instance. Also, the rear open ends 2a of the tubular bodies 2 may already be provided with a suitable fitting RC so that a fluid can be present in the interior space, possibly before the removal of the membranes M. A fluid connection is allowed without any requirement for handling the external actuators/parts 11, 9, which means a single gesture (along arrow F, Fig. 3A) is sufficient to have the fluid communication, while a combination of gestures can be required for disconnection, thus preventing accidental disconnection.

Use of slides 80, in each connector 1, 101 of the connection system 100, 100', allow forming relatively flat flanges 3 since each slide may extend in a transverse plane YZ perpendicular to the longitudinal axis X. Moreover, mounting of a locking slider 10, 110 or of a sliding unlocking external part 9 may be made without complex fastening members. The locking slider 10, 110, possibly formed as a molded plastic part comprising a wide open locking plate, may be a flat piece (compact piece along longitudinal axis X).

The tubular body 2, the plug 15 and/or the sliding part(s) in the connectors 1 and 101 can be obtained by molding a plastic material. In particular, the tubular body 2 and the plug 15 can be formed of plastic materials biocompatible with biopharmaceutical substances, for example polypropylene, polyethylene, polycarbonate, polysulfone, polyvinylidene chloride, polyethylenimine. This may apply for the end fitting RC as well.

Referring to Figs 3b and 8, the fluid coupling components A and B can be identical, each provided in a connector 1, 101 of similar or identical structure. Each axial passage/access 30 may extend offset on a side of the opening O, which is opposite relative to the side where the inserting tab 4, 104 or similar insert extends. The opening O may be a central opening, so that it may provide an axial passage, through the flange 3 and extended by the tubular body 2.

The coupling ends or components A, B may be identical, each with the same female connecting member 31 and the same male connecting member 32, so that the connectors 1, 101 are genderless. Practically, the connectors 1, 101 such as illustrated in Figs 1 and 2, or in Fig. 7 may be identical assemblies, with the connector 101 positioned in the reverse direction and rotated 180° as compared to the connector 1. While Fig. 3A shows the coupling before removal of the membranes M, Fig. 7 illustrates a configuration after disconnection, in which the connectors 1 and 101 have been set in a closing state.

As illustrated in Fig. 7, the connectors 1, 101 that are retrieved after use may be in a closed state obtained immediately after disconnecting the connection system 100 or 100'. Since the plug 15 is already close to the junction plane PJ (see also Figs 2 and 3B showing the position of the plugs 15, adjacent to front opening plane in retracted state of the plugs), the return means/member 20 can be still energized in operation. As soon as the connecting members 31, 32 in the connectors are unlocked, each plug 15 is pushed to have the return member 20 reaching an extended (more stable) stable position with the plug 15 protruding outside and engaging the seating surface S.

When having a connector arrangement combining a membrane, a connector 1, 101 including a substantially planar flange front face F3 and a valve assembly VA, a single use aseptic connection system 100, 100' is obtained.

While illustrated embodiments show a valve assembly VA with a return member 20 distinct from a sealing member J1, other structures may be implemented, for instance using a spring energized seal or a suitable spring part including an annular contact surface for enabling an annular sealing contact around the plug 15. In variants, the plug 15 may be a plastic part including a rear spring part, so that the return effect is obtained without using a separate piece.

Any reference sign in the following claims should not be construed as limiting the claim. It will be obvious that the use of the verb "to comprise" and its conjugations does not exclude the presence of any other elements besides those defined in any claim. The word "a" or "an" preceding an element does not exclude the presence of a plurality of such elements.

## Claims

1. A connector arrangement for fluid connection, comprising:
- a connector (1; 101) including a tubular body (2) extending around a longitudinal axis (X) of the connector and a plug (15) movable inside the tubular body (2); and
- an external blocking part (12a; 12b) provided with a blocker (14) to retain the plug (15) at a retaining region (15f) of the plug, which is preferably a rear retaining region, so that the plug (15) is maintained in an open position inside the tubular body (2),
wherein the connector (1; 101) comprises:
- a flange (3) formed peripherally on the tubular body (2) around a central opening (O) opening at a front of the tubular body (2), the flange (3) comprising fixation means preferably provided with a male connecting member (32) protruding axially from a flange front face (F3);
- an aseptic attachment structure (5) including a connector front face and a membrane (M) that is removably attached to the connector front face to seal the central opening (O) while the plug (15) is in the open position;
wherein upon removal of the external blocking part (12a; 12b), the plug (15) reaches or has been set in a released state so as to be movable, from the open position, to a closing position in which the plug (15) is sealing the central opening (O),
wherein the plug (15) is provided with a plug axial surface (F15) located adjacent to or extending at the central opening (O), so that in the closing position the plug axial surface (F15) protrudes axially outside beyond the central opening (O), at a front of the connector (1; 101); and wherein the external blocking part (12a; 12b) is coupled to the membrane (M) and removable, preferably simultaneously, with the membrane (M) upon a radial pulling action.

2. The connector arrangement according to claim 1, wherein the tubular body (2) extends around the longitudinal axis (X) between a first open end (2a) and a second open end (2b) provided with the central opening (O),
wherein an interior space (7) of the tubular body (2) is accessible via the opening (O), the plug (15) being movable in the interior space (7), parallel to the longitudinal axis (X), and having a sealing position inside the interior space (7) when reaching the closing position.

3. The connector arrangement according to claim 2, comprising an elastic return member (20), preferably arranged in the interior space (7), biasing the plug (15) toward the closing position,
wherein the connector (1; 101) comprises a valve assembly (VA) including:
- the plug (15);
- the tubular body (2) that defines an annular seating surface (S) in contact with the plug (15) in the closing position; and
- the elastic return member (20).

4. The connector arrangement according to claim 2 or 3, wherein the external blocking part (12a; 12b) comprises a first clamping member (6) for outer fixation to the tubular body (2) and a second clamping member including two branches (14b) forming the blocker (14), the two branches (14b) being engaged with the retaining region (15f) of the plug (15), which extends axially rearward relative to a sealing annular contact between an inner face of the tubular body (2) and a circumferential outer part of the plug (15),
and wherein the two branches (14b) directly contact the plug (15).

5. The connector arrangement according to claim 4, wherein the external blocking part (12a; 12b) is provided with a radial portion extending parallel to a transverse plane (YZ) perpendicular to the longitudinal axis (X), the radial portion being preferably radially spaced from the tubular body (2) by a radial gap.

6. The connector arrangement according to any one of the claims 1-5, wherein the fixation means include two connecting members (31, 32), which comprise a female connecting member (31) and a male connecting member (32), distributed on either side of the membrane (M).

7. The connector arrangement according to any one of the claims 1-5, wherein the fixation means comprise two connecting members (31, 32),
wherein the two connecting members (31, 32) comprise a female connecting member (31) and a male connecting member (32) that is protruding axially from the flange front face (F3),
and wherein the flange (3) is provided with a retaining rim or surface to lock an inserted position of an insert passing through an axial access (30) provided in the female connecting member (31).

8. The connector arrangement according to claim 7, wherein the male connecting member (32) comprises a first insert with a fastening region (4r), preferably under the form of a groove (G4) substantially perpendicular to the longitudinal axis (X);
wherein the flange (3) delimits a housing (8) or peripheral region extending around the interior space (7), the axial access (30) forming an access to the housing (8) or peripheral region and allowing insertion of a second insert of same structure as the first insert, the second insert including a fastening region (104r), the second insert being formed in a complementary connector (101; 1) that is suitable to be coupled to said connector (1; 101) for enabling the fluid connection;
wherein the retaining rim or surface, forming all or part of latching means (LM; LM'), is:
- arranged inside the housing (8) or at the peripheral region; and
- configured to retain the second insert in a locking configuration of the fluid connection by engaging the fastening region (104r) of the second insert, the locking configuration being obtained when the second insert is in the inserted position and positioned inside the female connecting member (31) via the axial access (30).

9. The connector arrangement according to claim 7 or 8, wherein the latching means (LM) include a piece distinct from the flange (3) and are operable by a manual actuation using an external actuator (11) or push-button (9) arranged to externally cover the flange (3) and/or radially protrude outward relative to the flange (3).

10. The connector arrangement according to claim 9, wherein the manual actuation causes an interfering portion (51) of the latching means (LM) to be moved radially away from an axial direction passing through the axial access (30) of the female connecting member (31).

11. The connector arrangement according to claim 9 or 10, wherein the external blocking part (12a; 12b) is overlapping the flange (3) on a first flange side,
wherein the external actuator (11) or push-button (9) is pushable at a second flange side that is perpendicular to the first flange side.

12. The connector arrangement according to any one of the preceding claims, wherein the housing (8) forms a slide (80) extending generally within a transverse plane (YZ) perpendicular to the longitudinal axis (X),
wherein the retaining rim or surface is included in a locking slider (10; 110) distinct from the tubular body (2) and inserted in the housing (8), the locking slider (10) being configured to perform the retaining of the second insert (104) at the fastening region (104r) of the second insert (104).

13. The connector arrangement according to claim 12, wherein the locking slider (10; 110) is annular, the retaining rim or surface being provided at an end of the locking slider (10) at the opposite from another end provided with an external actuator (11) that is pushable along a first direction (Y), which is an unlocking direction opposite to an elastic return force provided by the locking slider (10; 110).

14. The connector arrangement according to any one of the preceding claims when depending on claim 3, wherein the plug (15) is slidable on a sliding guide (34) provided with an abutment surface, so that the elastic return member (20) biases the plug (15) along a given direction of a biasing force, the plug (15) being configured to be axially blocked by engagement of at least one relief (R) with the abutment surface when the plug (15) is in the closing position, so that the plug (15) is prevented to be pushed against the biasing force exerted by the elastic return member (20).

15. A connection system (100; 100') comprising a first connector arrangement with a first connector (1) and a second connector arrangement with a second connector (101), which are each a connector arrangement as defined in any one of the preceding claims, the connection system (100; 100') allowing fluid connection in a coupled state obtained by coupling parts (A, B) distributed in the flange (3) of the first connector (1) and in the flange (3) of the second connector (101), using the fixation means (31, 32) to obtain a front-to-front or flange-to-flange connection between the connectors (1; 101) that have same longitudinal axis (X) in the coupled state,
wherein the external blocking part (12a) of the first connector (1) and the external blocking part (12b) of the second connector (101) are:
- each provided with a blocker (14) engaged inside the tubular body to retain the corresponding plug (15) in a retracted open position inside the tubular body (2),
- linked to form a clip assembly (12), the clip assembly (12) extending parallel to the longitudinal axis (X) and including a radial graspable portion (GP);
- configured to be simultaneously removed when pulling portions of the membranes (M), which are overlapping radial portions of the membrane interposed between the external blocking parts (12a; 12b) and offset relative to a covering region where each membrane (M) axially covers a corresponding central opening (O), the radial portions of the membranes (M) extending parallel to the radial graspable portion (GP),
and wherein the plugs (15) are each provided with one or more openings (O15) and are biased toward a junction plane (PJ) of the connection system by return means (20), so that in the coupled state, the plug axial surfaces (F15) are configured to be in mutual contact as soon as the membranes (M) and the clip assembly (12) have been removed, without preventing fluid to flow between the one or more openings (O15) of the plug of the first connector (1) and the one or more openings (O15) of the plug of the second connector (101).

16. The connection system according to claim 15, wherein the first and second connectors (1, 101) have identical structure, the first and second connectors (1, 101) being identical with only a difference of 180° in angular position, in the coupled state, for having the radial portions of the membranes (M) extending in same direction.

17. A method of assembling an arrangement with a fluid connector (1; 101), the method comprising:
- inserting a plug (15) inside a tubular body (2) via a first open end (2a) of the tubular body, which is a tubular body rear end;
- clipping an external blocking part (12a; 12b) around the tubular body (2) and engaging, on a retaining region (15f) of the plug, a blocker (14) that is integral with the external blocking part (12a; 12b), to retain the plug (15) in an open position inside the tubular body (2),
- covering a second open end (2b) of the tubular body, which is a tubular body front end, by a membrane (M), in order to seal a central opening (O) of the tubular body (2) and form an aseptic attachment structure (5), at least one connecting member being provided on a flange (3) formed peripherally on the tubular body (2), the at least one connecting member (31, 32) being offset relative to the membrane (M), the membrane (M) extending radially, to provide a pull portion, along a first transverse direction (Z) that is perpendicular to a longitudinal axis (X) passing through the central opening (O);
- coupling the external blocking part (12a; 12b) to the membrane (M), so that a pulling action for removing the membrane (M) can simultaneously drive an unclipping of the external blocking part (12; 12b);
wherein the plug (15) is mounted slidable inside the tubular body (2), so that upon removal of the external blocking part (12a; 12b) by the unclipping, the plug (15) reaches a released state to be movable, from the open position to a closing position in which:
- the plug (15) is sealing the central opening (O),
- a plug axial surface (F15) of the plug (15) protrudes axially outside beyond the central opening (O).

18. Use of a clip assembly (12) in the connection system (100; 100') of claim 15 or 16, wherein the clip assembly (12) is:
- preliminary clipped around each of the first connector (1) and the second connector (101), to simultaneously retain the plug of the first connector (1) and the plug of the second connector (101) in a position distal from a seating region surrounded by at least one annular seal (J) that is adjacent to the junction plane (PJ);
- arranged to pinch the membranes (M) at the graspable radial portion (GP), where the external blocking parts (12a, 12b) are mutually rigidly fastened;
and wherein unclipping of the clip assembly (12) simultaneously causes:
the membranes (M) to be removed by a radial pulling,
and the blockers (14) to be disengaged from the plugs (15).
